# EUROPEAN PATENT APPLICATION

(11) **EP 4 124 344 A1**
(43) Date of publication of application: **01.02.2023**
(21) Application number: 20914878.2
(22) Date of filing: 30.10.2020
(51) Int. Cl.: A61K 45/00, A61K 31/7088, A61K 31/713, A61K 39/395, A61K 48/00

(54) **NOVEL USE OF HIC-5 INHIBITOR**

(30) Priority: 20.01.2020 JP 2020007072; 10.07.2020 JP 2020119476
(71) Applicant: Showa University, Tokyo 142-8555 (JP)
(72) Inventor: KANEYAMA, Shuri, Tokyo 142-8555 (JP); LEI Xiao-Feng, Tokyo 142-8555 (JP)
(74) Representative: J A Kemp LLP
(86) International application number: PCT/JP2020/041644
(87) International publication number: WO 2021/149329

(57) **Abstract**

Provided is a method for treating a disease in which Hic-5 participates. A composition comprising a Hic-5 inhibitor, said composition being for treating a disease selected from among cardiomegaly, interstitial pneumonia, osteoarthritis, fatty liver and tumor, or for inhibiting vascular invasion of cancer cells.

## Description

### TECHNICAL FIELD

The present disclosure relates to a novel use of a Hic-5 inhibitor, in particular, to a treatment of a disease selected from cardiac hypertrophy, interstitial pneumonia, osteoarthritis, NASH, tumor, and the like, and a suppression of a vascular invasion of a cancer cell, etc., by a Hic-5 inhibitor.

### BACKGROUND ART

Hydrogen peroxide-inducible clone-5 (Hic-5) is a cell focal adhesion constituent molecule that belongs to the paxillin family. Cell focal adhesions are cellular machineries which connect extracellular matrix (ECM) and intracellular actin skeleton and are constituted by integrin and a plurality of focal adhesion proteins including Hic-5. The cell focal adhesions play an important role in cell adhesion, cell motility, morphological change of cells, and stimulus transmission to cells from surrounding environments, etc. Research using Hic-5 knockout mice, etc., has suggested the possibility that Hic-5 is involved in various diseases (Non-Patent Literatures 1 to 7). Treatment of scar formation has also been attempted by the inhibition of Hic-5 (Patent Literature 1).

### CITATION LIST

### Patent Literature

Patent Literature 1: US 2010/0292300 A1

### Non-Patent Literature

Non-Patent Literature 1: Kim-Kaneyama et al., J Atheroscler Thromb. 2012;19(7):601-7
Non-Patent Literature 2: Omoto et al., Oncogene. 2018;37(9): 1205-1219
Non-Patent Literature 3: Petropoulos et al., J Cell Biol. 2016;213(5):585-99
Non-Patent Literature 4: Lei et al., J Hepatol. 2016;64(1):110-7
Non-Patent Literature 5: Jamba et al., PLoS One. 2015;10(4):e0122773
Non-Patent Literature 6: Arita-Okubo et al., Cardiovasc Res. 2015;105(3):361-71
Non-Patent Literature 7: Yund et al., J Mol Cell Cardiol. 2009;47(4):520-7

### DISCLOSURE OF THE INVENTION

Further elucidation of diseases involving Hic-5 is expected.

Some embodiments of the present disclosure provide the following.
[1] A composition for use in the treatment of a disease selected from cardiac hypertrophy, interstitial pneumonia, osteoarthritis, fatty liver and tumor, comprising a Hic-5 inhibitor.
[2] The composition for use according to [1], wherein the cardiac hypertrophy is caused by an increased left ventricular load.
[3] The composition for use according to [1], wherein the cardiac hypertrophy is a compensated cardiac hypertrophy.
[4] A composition for use in suppressing a vascular invasion of a cancer cell, comprising a Hic-5 inhibitor.
[5] The composition for use according to any one of [1] to [4], wherein the Hic-5 inhibitor is selected from an inhibitory nucleic acid molecule, a genome editing molecule, and an anti-Hic-5 antibody or a nucleic acid molecule encoding the antibody.
[6] A method of detecting a disease selected from cardiac hypertrophy, interstitial pneumonia, osteoarthritis, fatty liver and cancer with a risk of vascular invasion, comprising:
   detecting an expression of Hic-5 in a tissue suspected of having the disease; and
   comparing the degree of expression of Hic-5 in the tissue suspected of having the disease with the degree of expression of Hic-5 in a normal tissue,
   wherein a greater degree of expression of Hic-5 in the tissue suspected of having the disease than the degree of expression of Hic-5t in the normal tissue indicates the presence of the disease.
[7] A composition for use in diagnosing a disease selected from cardiac hypertrophy, interstitial pneumonia, osteoarthritis, fatty liver and cancer with a risk of vascular invasion, comprising a Hic-5 detection reagent.

The present disclosure has revealed that Hic-5 is involved in diseases such as cardiac hypertrophy, osteoarthritis, interstitial pneumonia, fatty liver, and tumor, and the vascular invasion of cancer cells, and provides a novel therapeutic option for these diseases or pathological conditions.

### BRIEF DESCRIPTION OF DRAWINGS

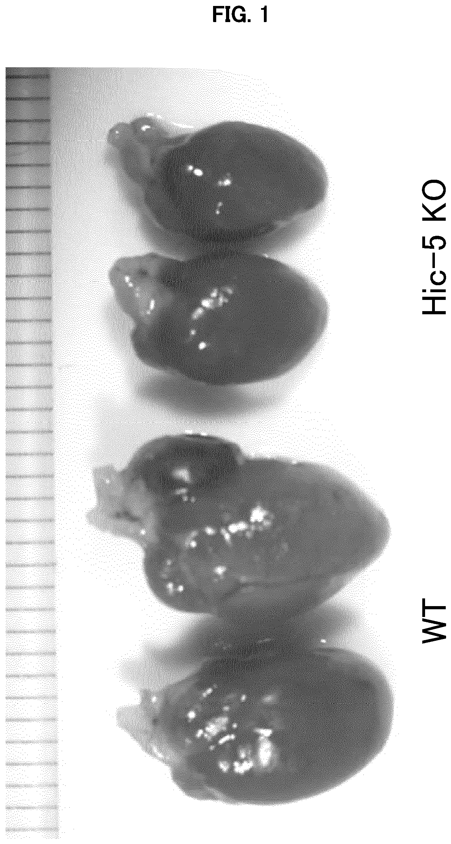

Figure 1 is photographs showing the appearance of the heart of Hic-5 KO mice (Hic-5 KO) and syngeneic wild-type mice (WT) that underwent TAC. The unit of the scale at the upper end is 1 mm.

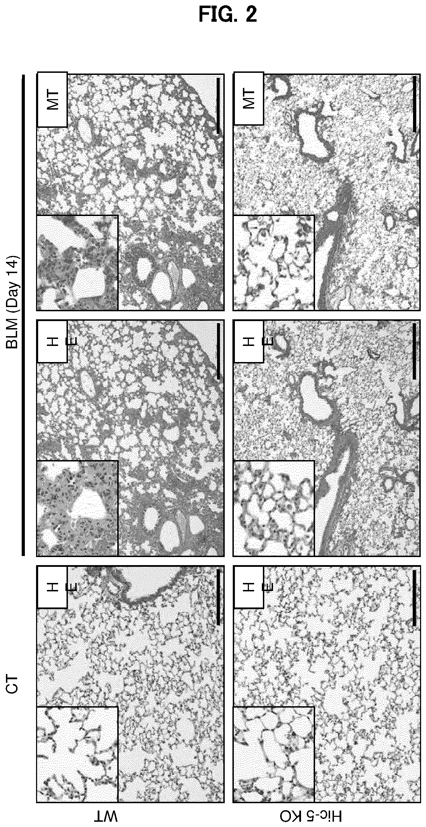

Figure 2 is optical microscopy images showing results of HE staining and Masson's trichrome (MT) staining of lung tissues from BLM interstitial pneumonia model mice. CT (left) and BLM (center and right) depict lung tissues on day 14 after BLM administration in BLM non-administration control group and BLM administration group, respectively. WT (upper) and Hic-5 KO (lower) depict lung tissues of wild-type mice and Hic-5 KO mice, respectively. The scale bars indicate 200 µm.

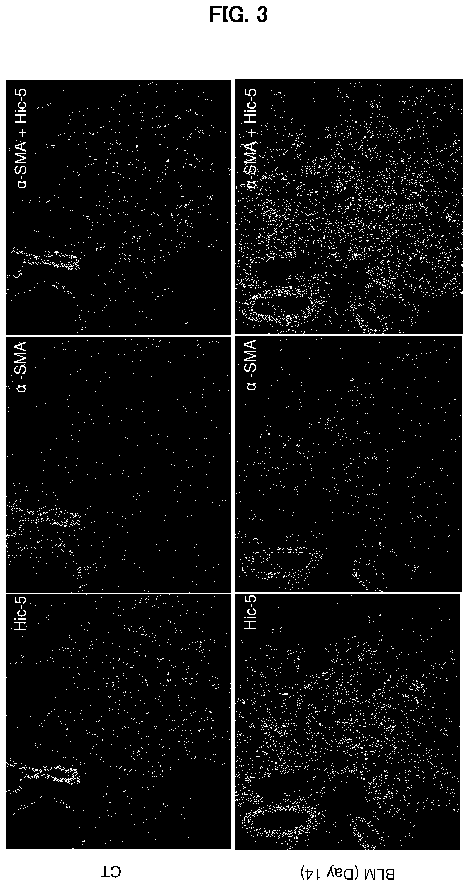

Figure 3 is fluorescence microscopy images showing results of immunostaining against Hic-5 and α-SMA of lung tissues from BLM interstitial pneumonia model mice (wild-type). The left images depict Hic-5, the central images depict α-SMA, and the right images depict merged Hic-5 and α-SMA. CT (upper) and BLM (lower) depict lung tissues on day 14 after BLM administration in BLM non-administration control group and BLM administration group, respectively.

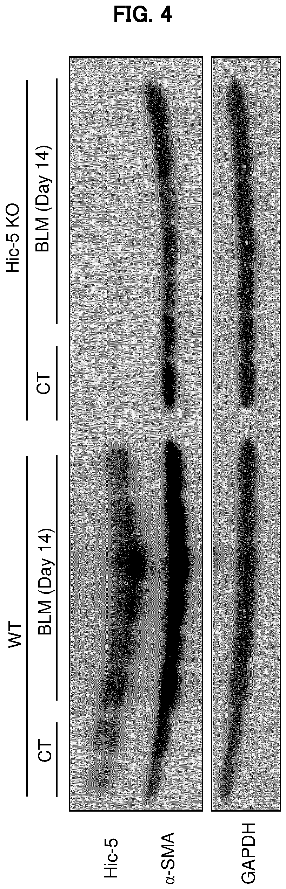

Figure 4 is a diagram showing results of evaluating Hic-5 and α-SMA in lung tissues of BLM interstitial pneumonia model mice by Western blotting. CT depicts BLM non-administration control group, and BLM depicts BLM administration group.

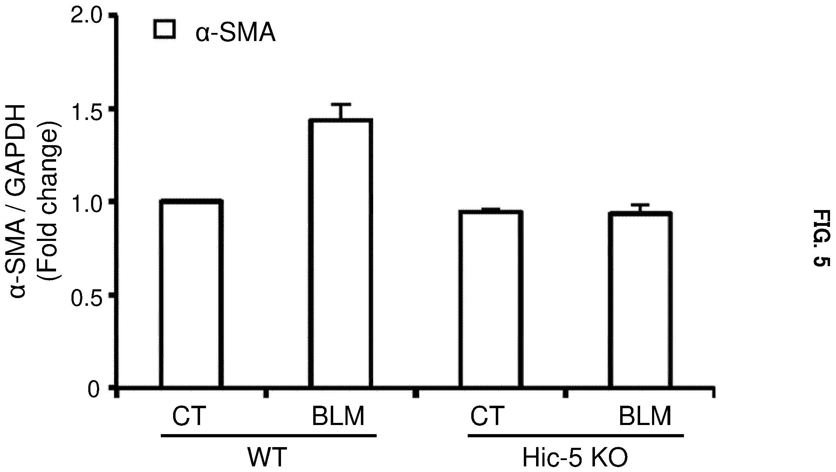

Figure 5 is a graph showing results of semi-quantifying Hic-5 and α-SMA in lung tissues of BLM interstitial pneumonia model mice by Western blotting. The ordinate shows fold changes in the expression level of Hic-5 or α-SMA based on the expression level of GAPDH.

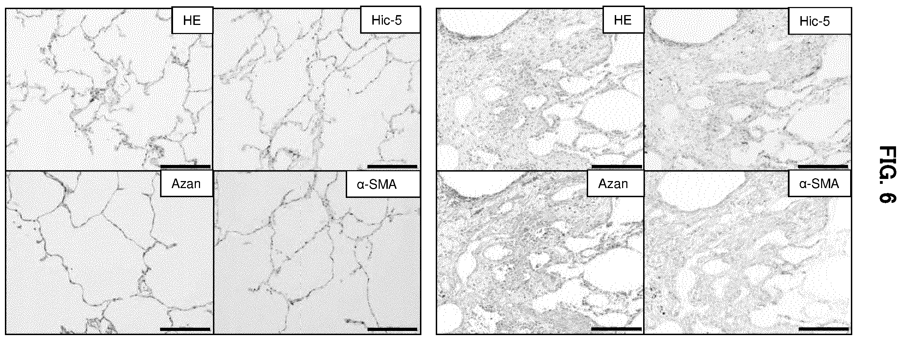

Figure 6 is microscopy images showing HE staining, Azan staining and immunostaining against Hic-5 or α-SMA of lung tissues from an interstitial pneumonia patient. The left images depict normal lung tissues, and the right images depict fibrotic lung tissues affected by interstitial pneumonia. The scale bars indicate 200 µm.

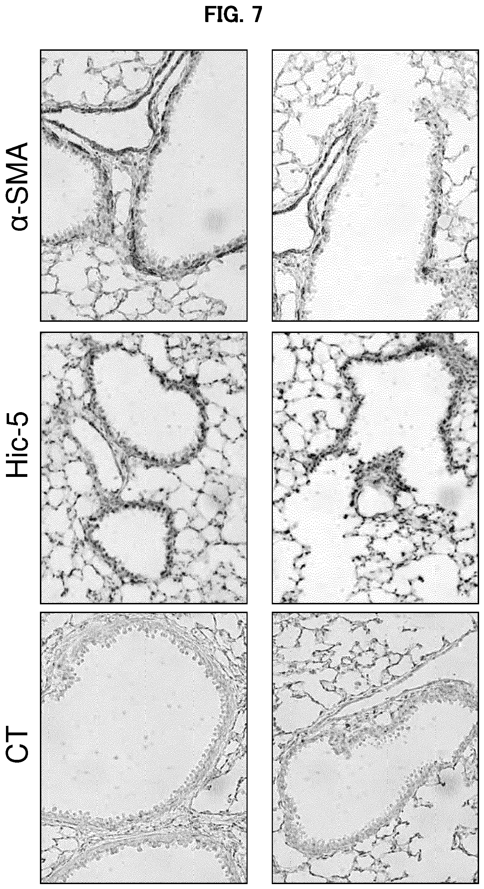

Figure 7 is representative microscopy images showing results of immunostaining against Hic-5 and α-SMA of lung tissues from BLM interstitial pneumonia model mice (wild-type). CT (left) depicts Hic-5 staining results for BLM non-administration control group, and Hic-5 (center) and α-SMA (right) depict Hic-5 and α-SMA staining results, respectively, for the lung tissues on day 14 after BLM administration in BLM administration group. In lung tissues of BLM administration group, Hic-5-positive and α-SMA-negative Clara cells were found around the bronchiole.

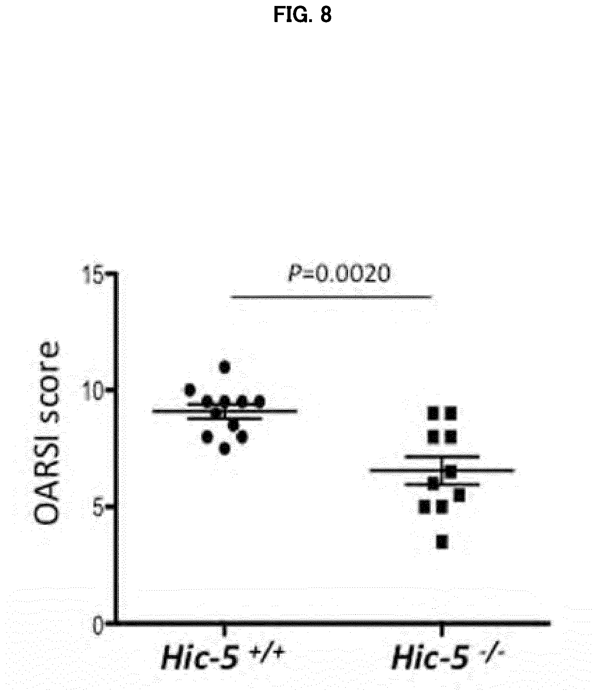

Figure 8 is a graph showing the OA severity of Hic-5 KO mice with induced osteoarthritis by OARSI scores (n = 11). Hic-5^{+/+} depicts wild type, and Hic-5^{-/-} depicts Hic-5 KO mice.

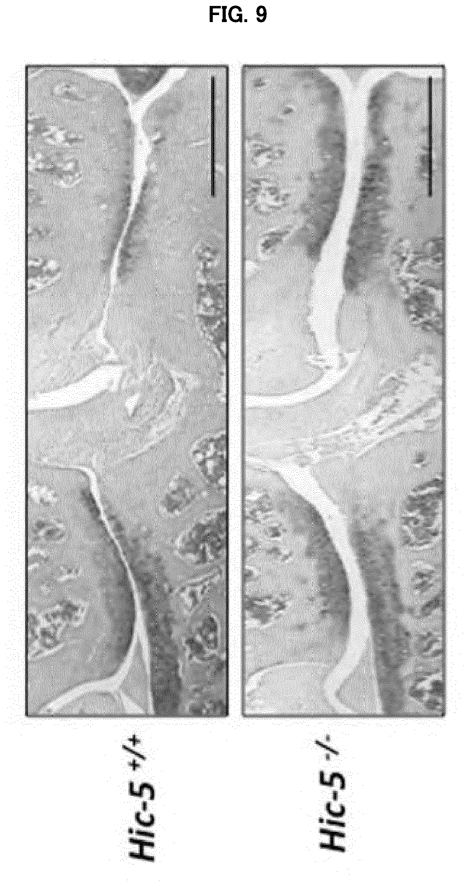

Figure 9 is optical microscopy images showing results of safranin-O staining of knee joint tissues from Hic-5 KO mice and wild-type mice with induced osteoarthritis. Hic-5^{+/+} depicts wild type, and Hic-5^{-/-} depicts Hic-5 KO mice. The scale bars indicate 200 µm.

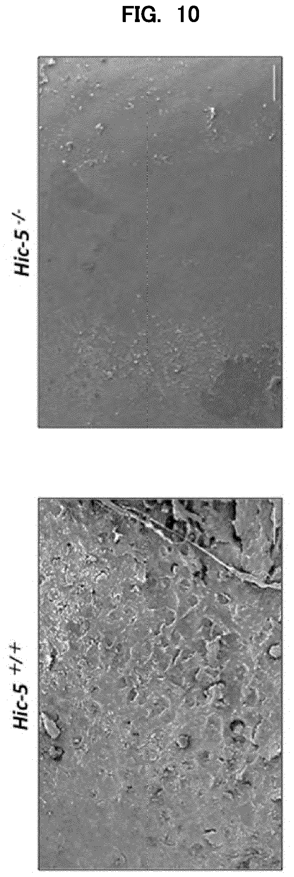

Figure 10 is scanning electron microscopy images of knee joint tissues of Hic-5 KO mice and wild-type mice with induced osteoarthritis. Hic-5^{+/+} depicts wild type, and Hic-5^{-/-} depicts Hic-5 KO mice. The scale bars indicate 20 µm.

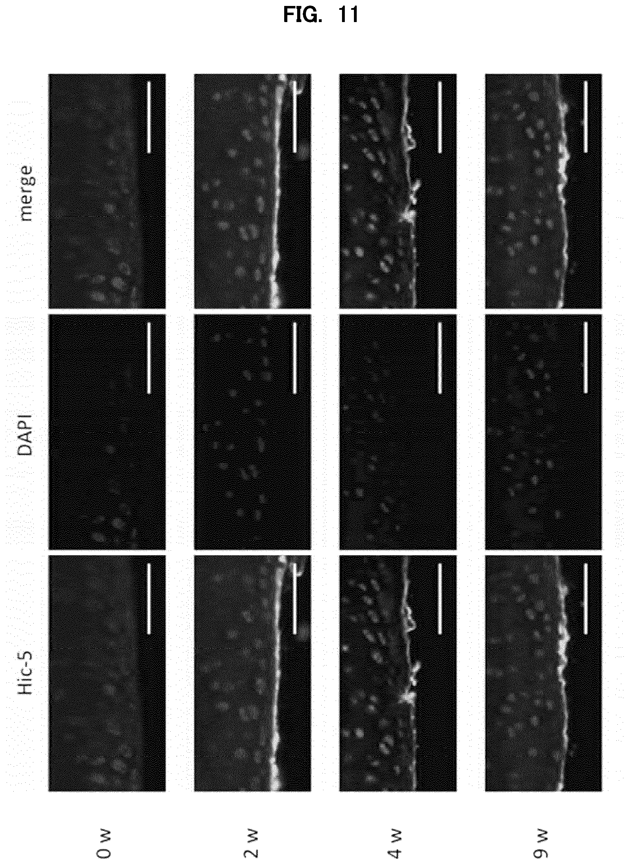

Figure 11 is fluorescence microscopy images showing results of immunostaining against Hic-5 of knee joint tissues from wild-type mice with induced osteoarthritis. The scale bars indicate 100 µm.

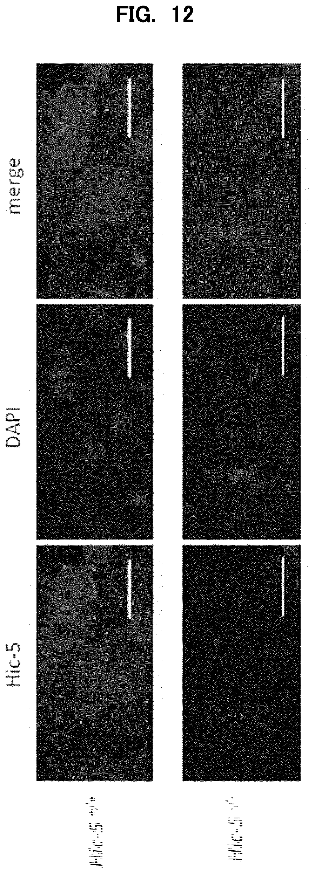

Figure 12 is fluorescence microscopy images showing the localization of Hic-5 in chondrocytes harvested from knee joints of Hic-5 KO mice and wild-type mice treated to induce osteoarthritis. Hic-5^{+/+} depicts wild type, and Hic-5^{-/-} depicts Hic-5 KO mice. The scale bars indicate 20 µm.

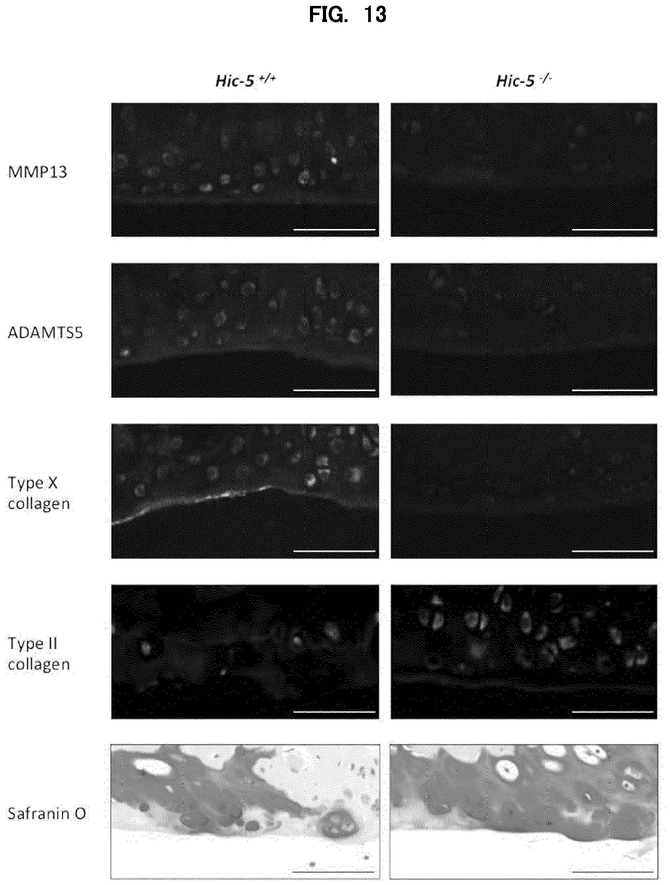

Figure 13 is fluorescence microscopy images showing the expression of MMP13, ADAMTS5, type II collagen and type X collagen in articular cartilage tissues of Hic-5 KO mice and wild-type mice treated to induce osteoarthritis, and safranin-O stain images of the same tissues thereas. Hic-5^{+/+} depicts wild type, and Hic-5^{-/-} depicts Hic-5 KO mice. The scale bars indicate 100 µm.

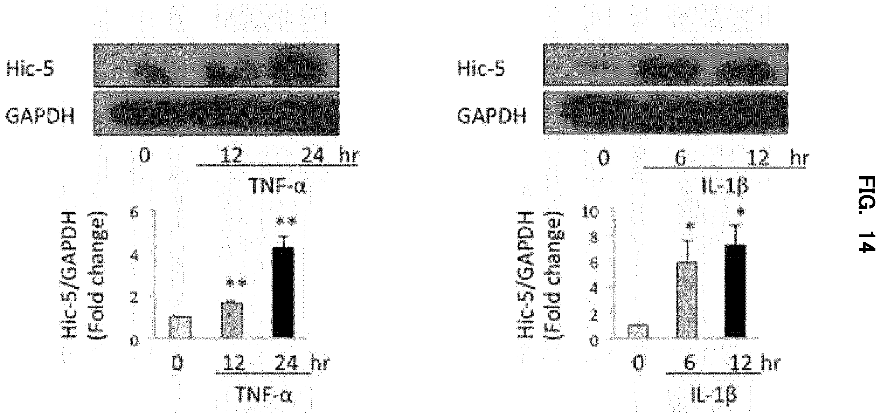

Figure 14 is a diagram showing how the expression level of Hic-5 in chondrocytes isolated from knee joints of wild-type mice would be changed due to TNF-a (left) or IL-1β (right). The upper columns show Western blot images, and the lower graphs show fold changes in the expression level of Hic-5 based on the expression level of GAPDH in the upper Western blot images (n = 3, mean ± SD, *P < 0.05, **P < 0.01).

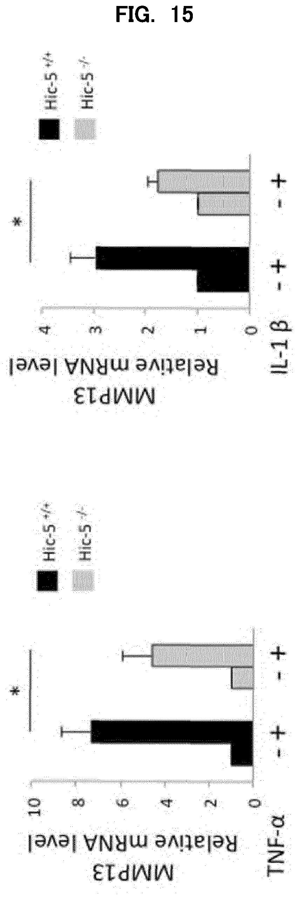

Figure 15 is a diagram showing how the expression level of MMP13 mRNA in chondrocytes isolated from knee joints of Hic-5 KO mice or wild-type mice would be changed due to TNF-a (left) or IL-1β (right) (n = 3, mean ± SD, *P < 0.05). Hic-5^{+/+} depicts wild type, and Hic-5^{-/-} depicts Hic-5 KO mice.

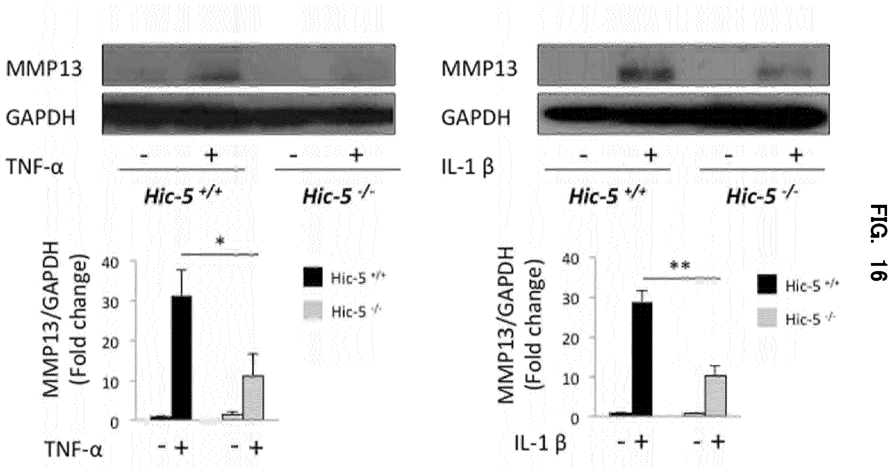

Figure 16 is a diagram showing how the expression level of MMP13 in chondrocytes isolated from knee joints of Hic-5 KO mice or wild-type mice would be changed due to TNF-a (left) or IL-1β (right). The upper columns show Western blot images, and the lower graphs show fold changes in the expression level of MMP13 based on the expression level of GAPDH in the upper Western blot images (n = 3, mean ± SD, *P < 0.05, **P < 0.01). Hic-5^{+/+} depicts wild type, and Hic-5^{-/-} depicts Hic-5 KO mice.

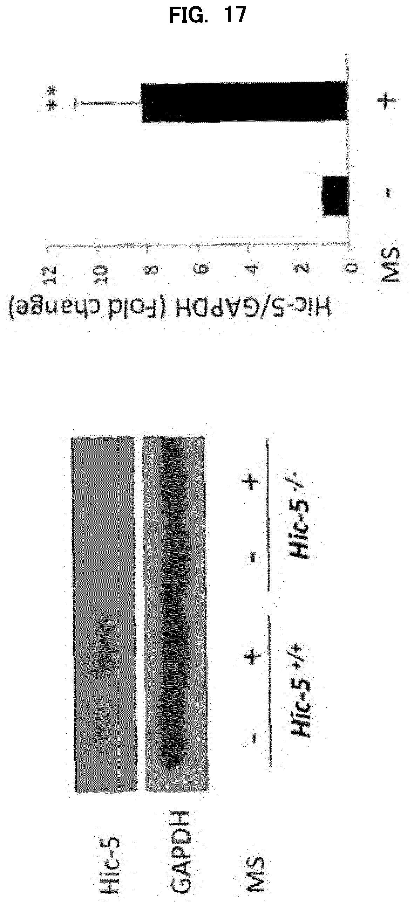

Figure 17 is a diagram showing how the expression level of Hic-5 in chondrocytes isolated from knee joints of Hic-5 KO mice or wild-type mice would be changed due to mechanical stress (MS). Western blot images are shown on the left side, and the right graphs show fold changes in the expression level of Hic-5 in the wild-type mice based on the expression level of GAPDH in the left Western blot images (n = 5, mean ± SD, **P < 0.01). Hic-5^{+/+} depicts the wild type, and Hic-5^{-/-}depicts the Hic-5 KO mice.

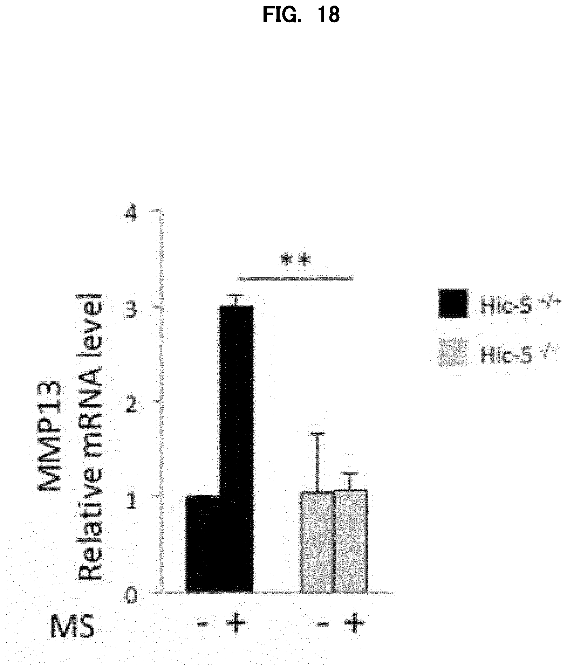

Figure 18 is a diagram showing how the expression level of MMP13 mRNA in chondrocytes isolated from knee joints of Hic-5 KO mice or wild-type mice would be changed due to mechanical stress (MS) (n = 3, mean ± SD, *P < 0.05). Hic-5^{+/+} depicts wild type, and Hic-5^{-/-} depicts Hic-5 KO mice.

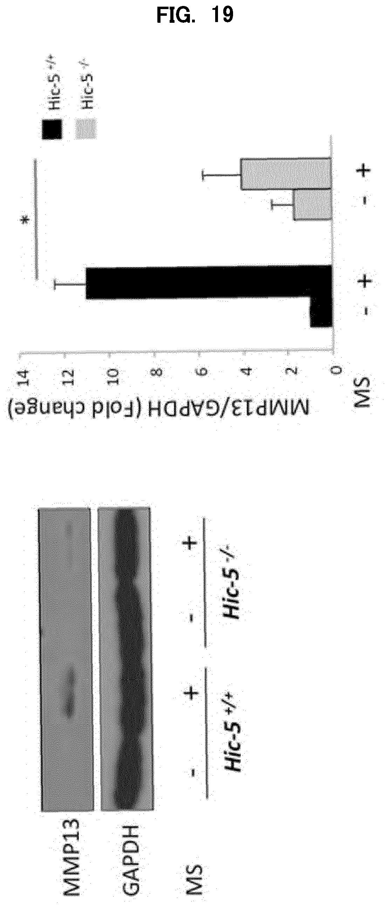

Figure 19 is a diagram showing how the expression level of MMP13 in chondrocytes isolated from knee joints of Hic-5 KO mice or wild-type mice would be changed due to TNF-α or IL-1β. Western blot images are shown on the left side, and the right graphs show fold changes in the expression level of MMP13 based on the expression level of GAPDH in the left Western blot images (n = 3, mean ± SD, *P < 0.05). Hic-5^{+/+} depicts wild type, and Hic-5^{-/-} depicts Hic-5 KO mice.

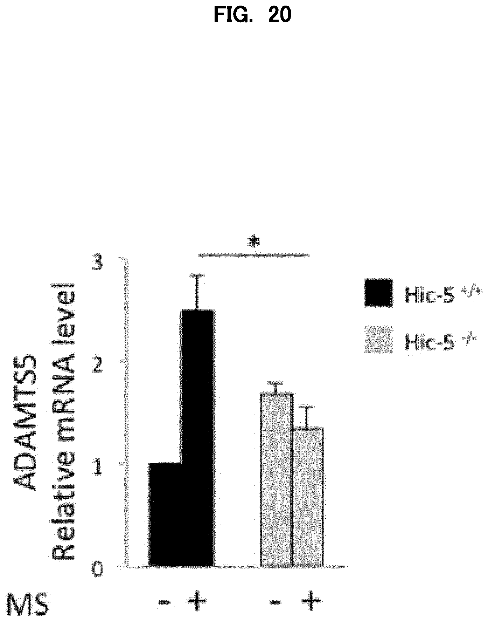

Figure 20 is a diagram showing how the expression level of ADAMTS5 mRNA in chondrocytes isolated from knee joints of Hic-5 KO mice or wild-type mice would be changed due to mechanical stress (MS) (n = 3, mean ± SD, *P < 0.05). Hic-5^{+/+} depicts wild type, and Hic-5^{-/-} depicts Hic-5 KO mice.

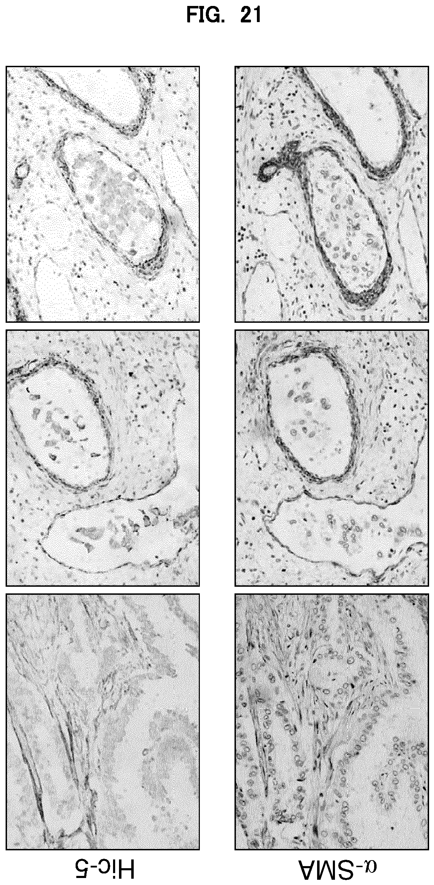

Figure 21 is microscopy images showing results of immunostaining against Hic-5 (upper) or α-SMA (lower) of lung tissues from a lung adenocarcinoma patient.

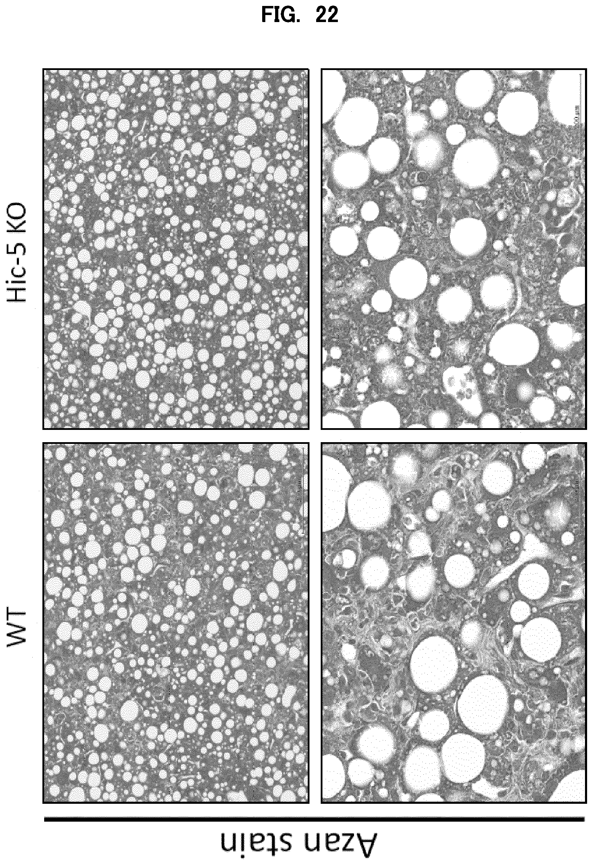

Figure 22 is microscopy images showing results of Azan staining of liver tissues from wild-type (WT) mice and Hic-5 KO mice with induced NASH. Similar level of fat droplet deposition was confirmed in both mice (upper). As is evident, liver tissues of wild-type mice had marked fibrosis, whereas fibrosis was rarely found in Hic-5 KO (lower).

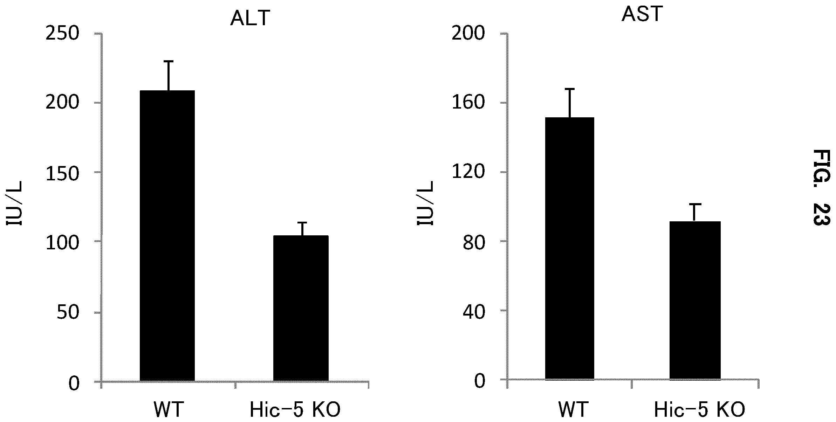

Figure 23 is graphs showing hepatocellular injury markers ALT and AST in serum of wild-type mice (WT) and Hic-5 KO mice with induced NASH.

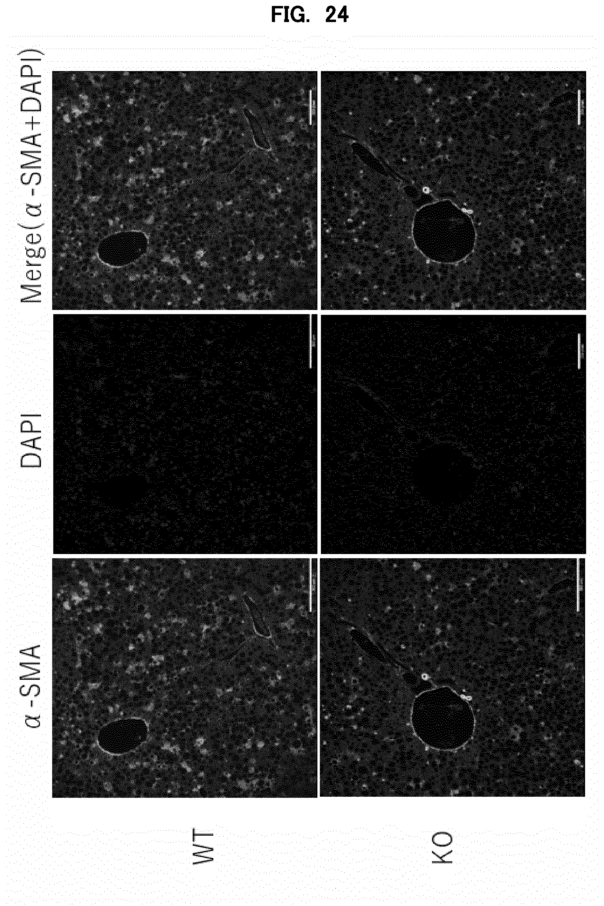

Figure 24 is fluorescence microscopy images showing results of immunostaining against α-SMA of liver tissues from wild-type (upper) mice and Hic-5 KO mice (lower) with induced NASH. The left images depict α-SMA, the central images depict DAPI, and the right images depict merged α-SMA and DAPI. The scale bars indicate 300 µm.

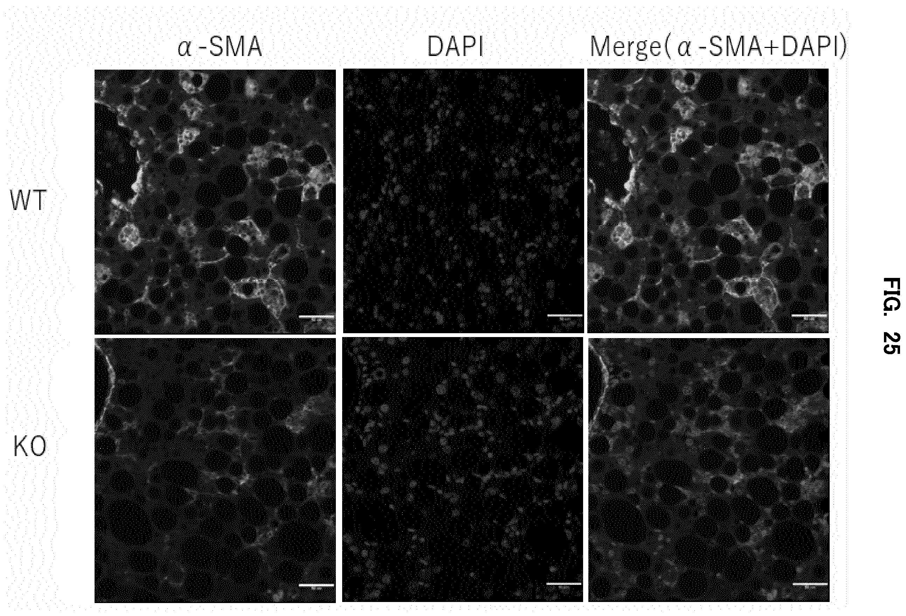

Figure 25 is fluorescence microscopy images showing results of immunostaining against α-SMA of liver tissues from wild-type mice (upper) and Hic-5 KO mice (lower) with induced NASH. The left images depict α-SMA, the central images depict DAPI, and the right images depict merged α-SMA and DAPI. The scale bars indicate 50 µm.

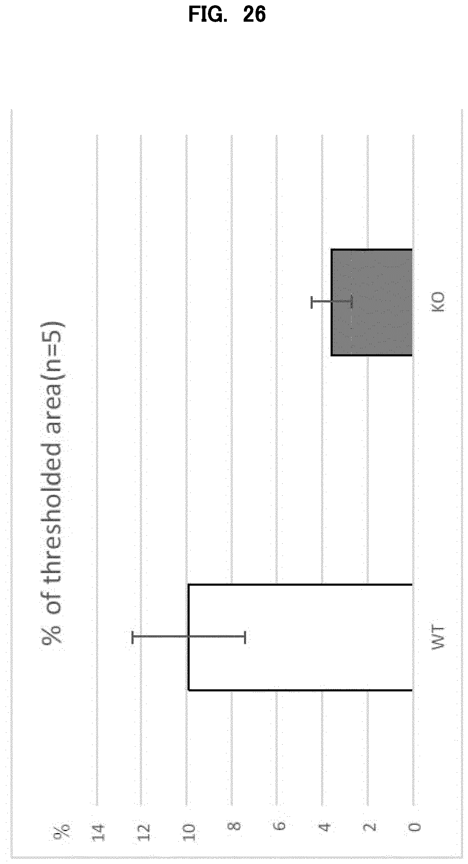

Figure 26 shows a graph in which the area ratio of α-SMA-positive cells in liver tissues was compared between wild-type (WT) mice and Hic-5 KO mice with induced NASH. KO depicts Hic-5 KO mice.

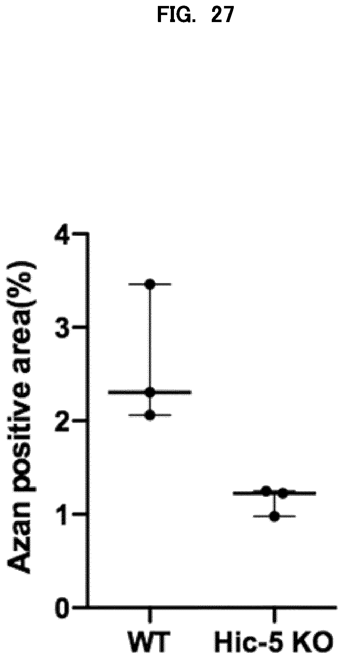

Figure 27 shows a graph in which the area ratio of regions stained blue by Azan staining in liver tissues was compared between wild-type mice (WT) and Hic-5 KO mice (Hic-5 KO) with induced NASH.

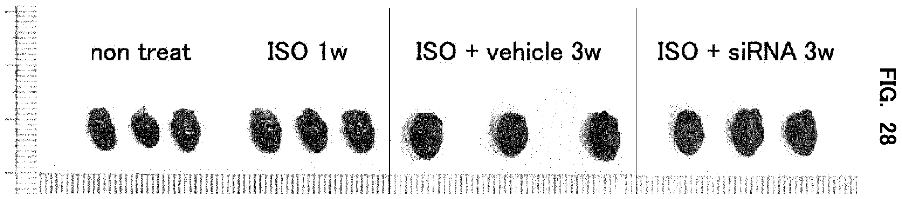

Figure 28 is a diagram showing the effect of Hic-5 inhibitor on mouse isoproterenol-induced cardiac hypertrophy models. "non treat" depicts untreated control group, "ISO 1w" depicts pump-treated mice on day 7, "ISO + vehicle 3w" depicts vehicle control group on day 30, and "ISO + siRNA 3w" depicts treatment group on day 30. The unit of the scale is 1 mm.

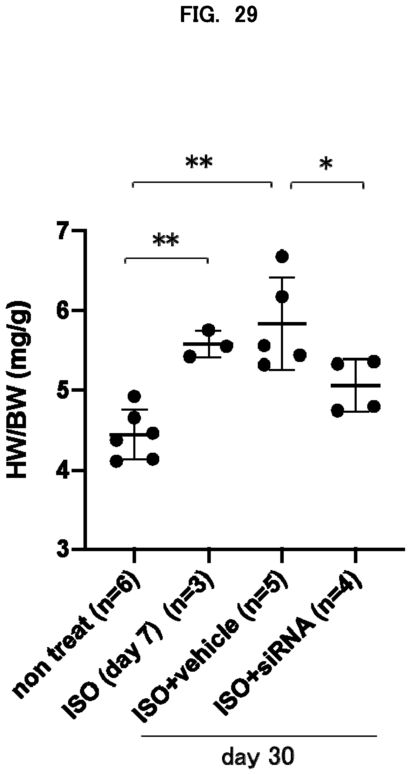

Figure 29 is a diagram showing the effect of Hic-5 inhibitor on mouse isoproterenol-induced cardiac hypertrophy models. "non treat" depicts untreated control group, "ISO (day 7)" depicts pump-treated mice on day 7, "ISO + vehicle" depicts vehicle control group on day 30, and "ISO + siRNA" depicts treatment group on day 30. The ordinate shows the ratio of the heart weight (mg) to the body weight (g) (HW/BW). *P < 0.05, **P < 0.01.

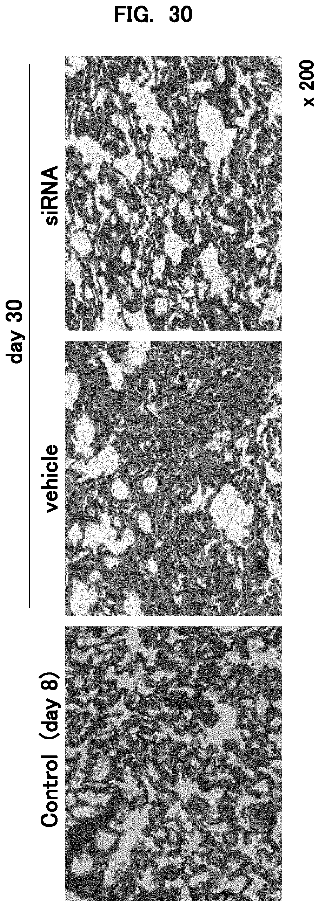

Figure 30 is optical microscopy images showing results of Masson's trichrome (MT) staining of lung tissues from BLM interstitial pneumonia model mice (200×). "Control" (left) depicts pretreatment control group, "vehicle" (center) depicts vehicle control group, and "siRNA" (right) depicts treatment group.

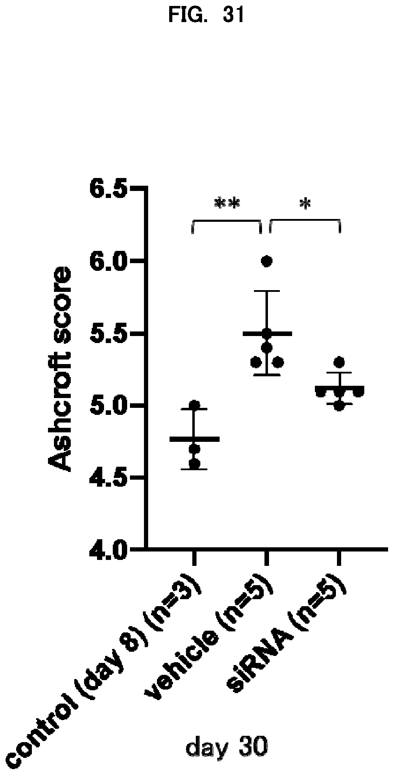

Figure 31 is a graph showing Ashcroft scores based on Masson's trichrome (MT) stain images of lung tissues from BLM interstitial pneumonia model mice. "control" depicts pretreatment control group, "vehicle" depicts vehicle control group, and "siRNA" depicts treatment group. *P < 0.05, **P < 0.01.

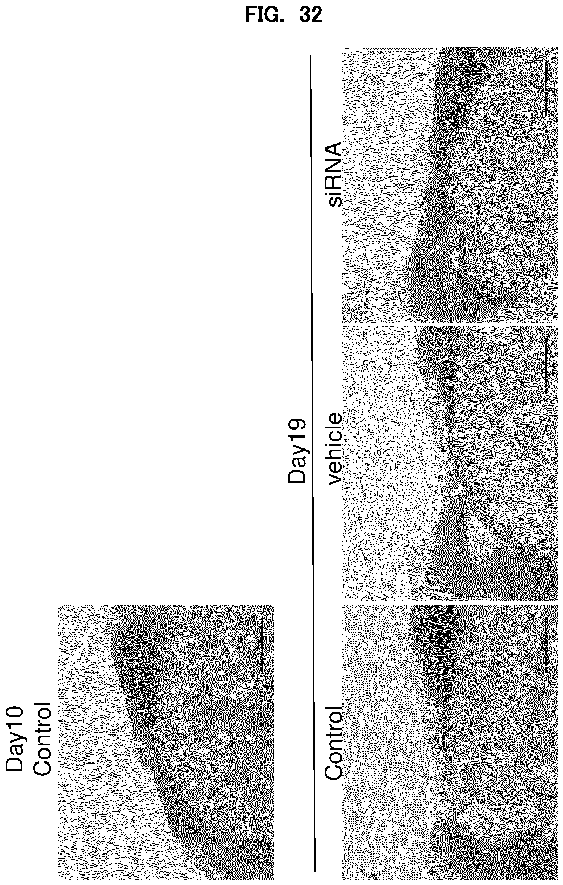

Figure 32 is representative safranin-O stain images of tibia tissues of each group. "Day10 Control" depicts preadministration control group, "Control" depicts untreated control group, "vehicle" depicts vehicle control group, and "siRNA" depicts siRNA administration group. The scale bars indicate 500 µm.

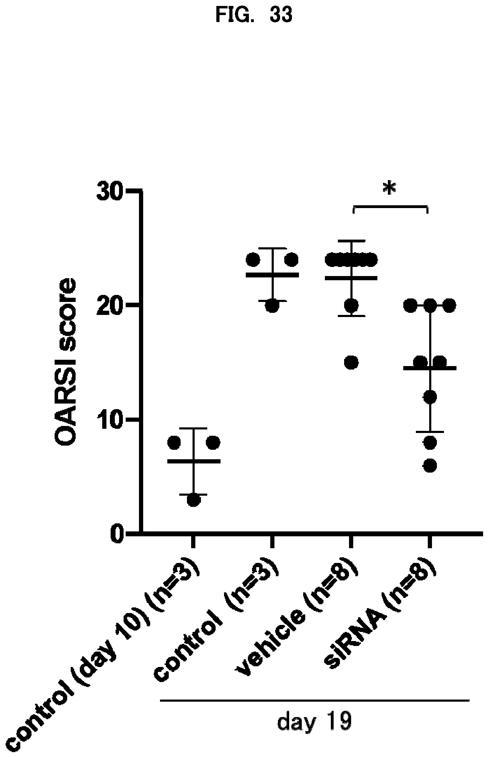

Figure 33 is a graph showing the OARSI score of tibia of each group. "Control (day 10)" depicts preadministration control group, "control" depicts untreated control group, "vehicle" depicts vehicle control group, and "siRNA" depicts siRNA administration group. *P < 0.05.

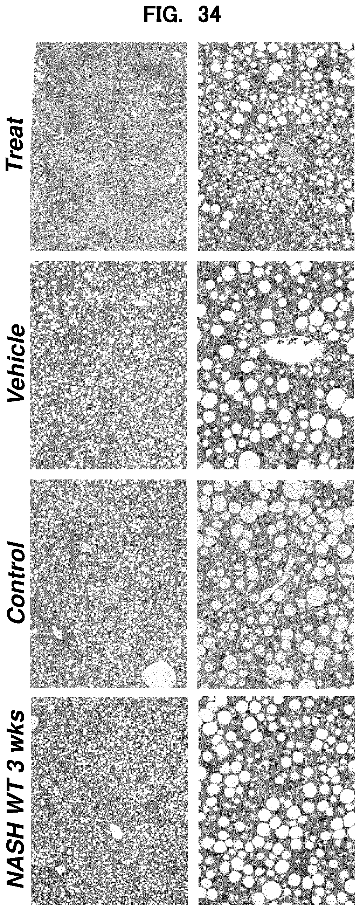

Figure 34 is a photographic diagram showing representative HE stain images of liver tissues of each group. "NASH WT 3 wks" depicts preadministration control group, "Control" depicts untreated control group, "Vehicle" depicts vehicle control group, and "Treat" depicts siRNA treatment group. The upper images were taken at a 50× magnification, and the lower images were taken at a 200× magnification.

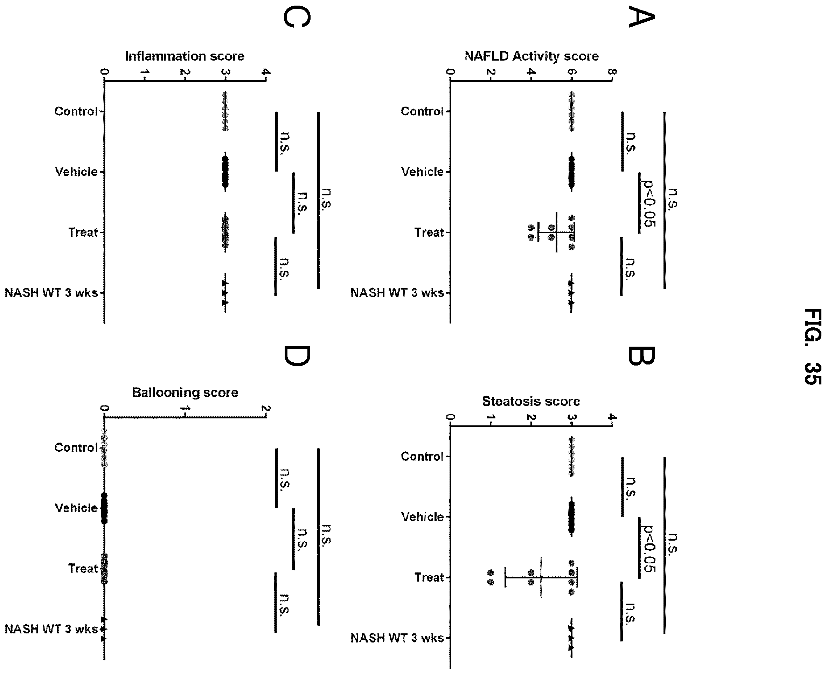

Figure 35 shows the NAS (A), steatosis score (B), inflammation score (C) and hepatocellular injury score (D) of each group. "NASH WT 3 wks" depicts preadministration control group, "Control" depicts untreated control group, "Vehicle" depicts vehicle control group, and "Treat" depicts siRNA treatment group.

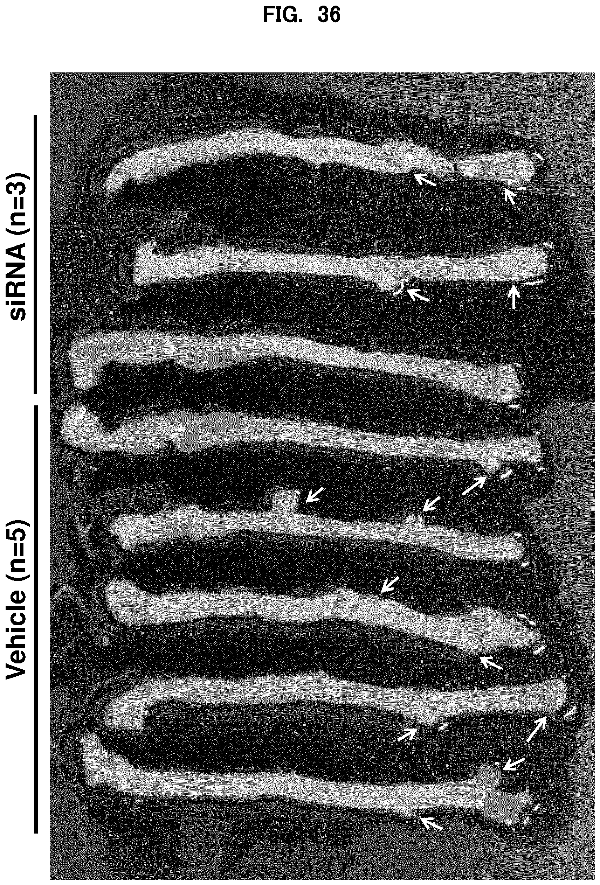

Figure 36 is a photographic diagram showing the appearance of large intestine of each individual 5 weeks after pump transplantation. "Vehicle" depicts vehicle control group, and "siRNA" depicts siRNA treatment group. The arrows show macroscopically confirmed colorectal tumor.

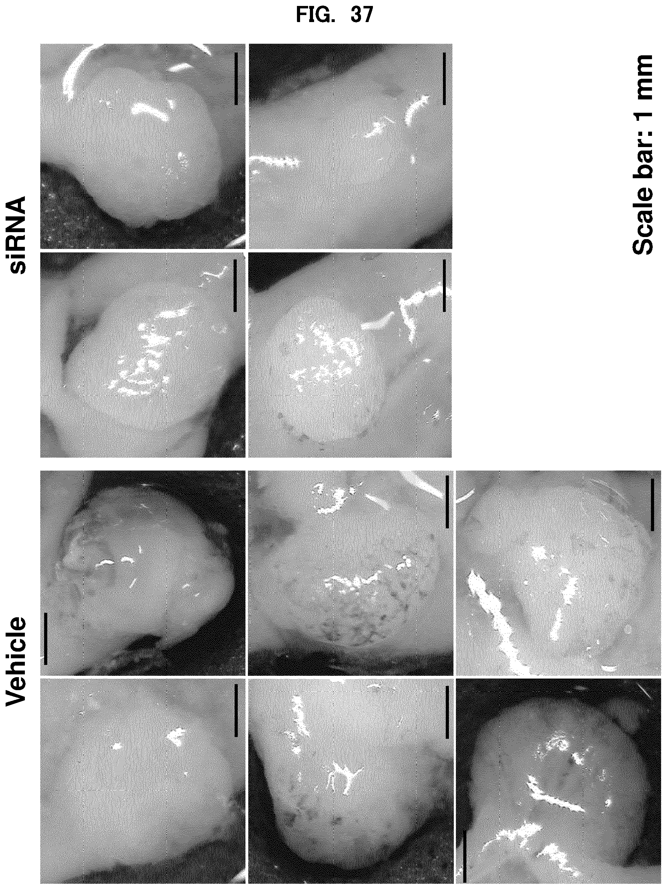

Figure 37 is a photographic diagram showing enlarged images of representative colorectal tumor confirmed 5 weeks after pump transplantation. "Vehicle" depicts vehicle control group, and "siRNA" depicts siRNA treatment group. The scale bars indicate 1 mm.

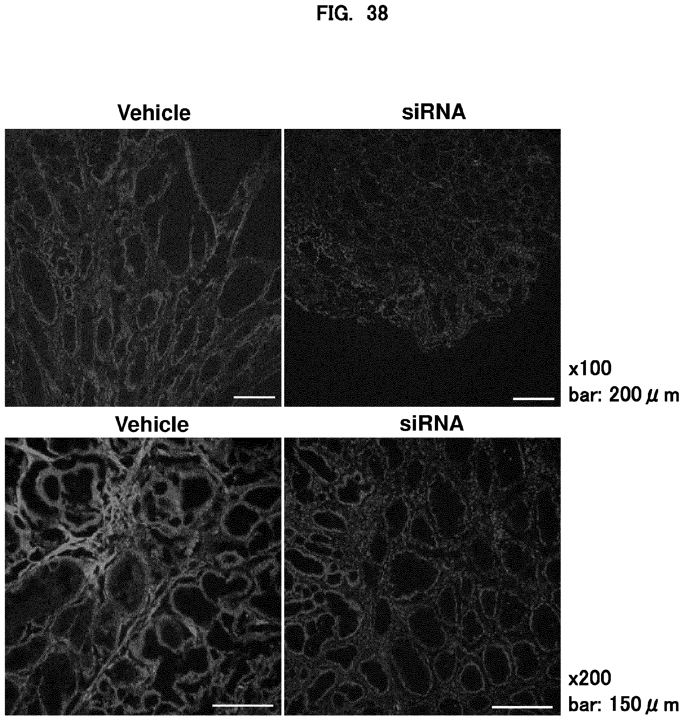

Figure 38 is a photographic diagram showing the expression of CD34 in representative colorectal tumor tissues of each group. "Vehicle" depicts vehicle control group, and "siRNA" depicts siRNA treatment group. The scale bars indicate 200 µm for the upper images (100× magnification) and 150 µm for the lower images (200× magnification).

### DESCRIPTION OF EMBODIMENTS

All technical terms and scientific terms used herein have the same meanings as those usually understood by those skilled in the art, unless otherwise specified herein. All patents, applications and other publications (including online information) cited herein are incorporated herein by reference in their entirety. The present application claims the priority benefit of the Japanese application filed on January 20, 2020 (Japanese Application No. 2020-007072) and the Japanese application filed on July 10, 2020 (Japanese Application No. 2020-119476), the contents described in the specification and drawings of which are incorporated herein.

In one embodiment, the present disclosure relates to a composition for use in the treatment of a disease selected from cardiac hypertrophy, interstitial pneumonia, osteoarthritis, fatty liver and tumor, comprising a Hic-5 inhibitor. Hereinafter, the composition may be referred to as the "composition of the present disclosure for use in the treatment" or the "composition A of the present disclosure". The composition for use in the treatment of cardiac hypertrophy, comprising a Hic-5 inhibitor may be referred to as the "composition of the present disclosure for use in the treatment of cardiac hypertrophy" or the "composition A1 of the present disclosure"; the composition for use in the treatment of interstitial pneumonia, comprising a Hic-5 inhibitor may be referred to as the "composition of the present disclosure for use in the treatment of interstitial pneumonia" or the "composition A2 of the present disclosure"; the composition for use in the treatment of osteoarthritis, comprising a Hic-5 inhibitor may be referred to as the "composition of the present disclosure for use in the treatment of osteoarthritis" or the "composition A3 of the present disclosure"; the composition for use in the treatment of fatty liver, comprising a Hic-5 inhibitor may be referred to as the "composition of the present disclosure for use in the treatment of fatty liver" or the "composition A4 of the present disclosure"; and the composition for use in the treatment of tumor, comprising a Hic-5 inhibitor may be referred to as the "composition of the present disclosure for use in the treatment of tumor" or the "composition A5 of the present disclosure".

In the present disclosure, the Hic-5 inhibitor means a substance that inhibits the expression and/or activity of Hic-5. Examples of the substance that inhibits the expression of Hic-5 include, but are not limited to, substances that modify Hic-5 gene, substances that inhibit the transcription of Hic-5 gene, substances that degrade transcripts of Hic-5 gene, substances that inhibit the translation of Hic-5 gene, and substances that inhibit normal folding of Hic-5 gene. Preferred examples of the substance that inhibits the expression of Hic-5 include inhibitory nucleic acids and genome editing molecules, particularly, inhibitory nucleic acids and genome editing molecules targeting Hic-5. Examples of the substance that inhibits the activity of Hic-5 include, but are not limited to, substances that inhibit the interaction between Hic-5 and another molecule, e.g., substances that bind to Hic-5, particularly, antibodies against the antigen Hic-5 and their antigen binding derivatives, and Hic-5 binding antibody mimetics. Other examples of the substance that inhibits the activity of Hic-5 include dominant negative mutants of Hic-5 (Shibanuma et al., Mol Biol Cell. 2003;14(3):1158-71). The Hic-5 inhibitor according to the present disclosure also includes a nucleic acid molecule encoding the aforementioned substance that inhibits the expression and/or activity of Hic-5.

The inhibitory nucleic acid molecule means a nucleic acid molecule that inhibits the expression of a target molecule and includes RNAi molecule, microRNA, microRNA mimic, piRNA (Piwi-interacting RNA), ribozyme (see, e.g., Jimenez et al., Trends Biochem Sci. 2015;40(11):648-661), antisense nucleic acids, and Bonac nucleic acids, for example.

The RNAi (RNA interference) molecule means any molecule having RNAi activity and encompasses, e.g., but is not limited to, siRNA (small interfering RNA), shRNA (short hairpin RNA), and the like. The RNA interference typically refers to a phenomenon, induced by a double-stranded nucleic acid molecule, in which target RNA is degraded in a sequence-specific manner. Upon entrance into a cell, the double-stranded nucleic acid molecule is cleaved by dicer according to its length and then, is incorporated into RNA-induced silencing complex (RISC) containing Argonaute (AGO) protein. RISC is guided by an antisense strand (guide strand) having a sequence complementary to target RNA so that the RISC recognizes and cleaves the target RNA.

siRNA typically refers to a small nucleic acid with an antisense strand having complementarity to a target sequence, and a sense strand having complementarity to the antisense strand, wherein both the strands at least partially form a duplex.

The antisense strand and the sense strand in siRNA may each independently have a length of 15 to 49 nucleotides (e.g., a length of 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49 nucleotides), 17 to 35 nucleotides, 17 to 30 nucleotides, 15 to 25 nucleotides, 18 to 25 nucleotides, 18 to 23 nucleotides, 19 to 21 nucleotides, 25 to 30 nucleotides, or 26 to 28 nucleotides. The duplex region may have a length of 15 to 49 nucleotides (e.g., a length of about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49 nucleotides), 15 to 35 nucleotides, 15 to 30 nucleotides, about 15 to 25 nucleotides, 17 to 25 nucleotides, 17 to 23 nucleotides, 17 to 21 nucleotides, 25 to 30 nucleotides, or 25 to 28 nucleotides.

In some embodiments, the sense strand and the antisense strand of siRNA are separate polynucleotide strands. In such embodiments, the antisense strand and the sense strand may form a double-stranded structure via a hydrogen bond, e.g., Watson-Crick base pairing, or through noncovalent linkage to each other. In other embodiments, the sense strand and the antisense strand constitute a portion of a single polynucleotide strand having a sense region and an antisense region. In such embodiments, the polynucleotide strand may have a hairpin structure.

siRNA may have a blunt end or a protruding end. The protruding end may have an overhang of 1, 2, 3, 4, 5, 6, 7 or 8 nucleotides. The overhang may be present at either the 5' end or the 3' end of the antisense strand and/or the sense strand or may be present at both the 5' end and the 3' end of the antisense strand or the sense strand. Specifically, the overhang may be present at the 5' end of the antisense strand, the 3' end of the antisense strand, both the 5' end and the 3' end of the antisense strand, the 5' end of the sense strand, the 3' end of the sense strand, both the 5' end and the 3' end of the sense strand, both the 5' end of the antisense strand and the 5' end of the sense strand, or both the 3' end of the antisense strand and the 3' end of the sense strand. The ends of siRNA may be symmetric or asymmetric. Examples of the siRNA having symmetric ends (hereinafter, may also be referred to as "symmetric siRNA") include siRNA having a blunt end at each end, and siRNA having overhangs on the same sides of the antisense strand and the sense strand (e.g., siRNA having overhangs with the same numbers of nucleotides at both the 5' end of the antisense strand and the 5' end of the sense strand, and siRNA having overhangs with the same numbers of nucleotides at both the 3' end of the antisense strand and the 3' end of the sense strand). Examples of the siRNA having asymmetric ends (aiRNA) include siRNA having a blunt end at one of the ends and a protruding end at the other end, and siRNA having protruding ends at both ends which however differ in the position, length and/or type of an overhang. Examples of the siRNA having asymmetric ends, both of which are protruding ends include siRNA having overhangs at both the 5' end and the 3' end of the antisense strand or the sense strand, and siRNA having overhangs on the same sides (i.e., the 5' ends or the 3' ends) of the antisense strand and the sense strand which however differ in the length and/or type of an overhang. The difference in the type of an overhang means, e.g., difference in the types of nucleotides constituting the overhang. The nucleotides constituting the overhang include RNA, DNA, and nucleic acids having various modifications mentioned later. Thus, an overhang constituted by only unmodified RNA differs in type from an overhang comprising modified RNA, and an overhang constituted by certain modified RNA differs in type from an overhang constituted by another modified RNA.

In another embodiment, siRNA may have a terminal loop structure. For example, siRNA may have a hairpin structure having a loop structure at one of the ends and a blunt end at the other end (one polynucleotide has the sense strand and the antisense strand) or may have a hairpin structure having a loop structure at one of the ends and a protruding end at the other end (e.g., having an overhang of 1, 2, 3, 4, 5, 6, 7 or 8 nucleotides). In the latter case, the overhang may be a 3' overhang or a 5' overhang, and the overhang may be present in the sense strand or the antisense strand.

In some embodiments, the sense strand of siRNA may comprise one or more nicks (e.g., sisiRNA). In such embodiments, the sense strand is divided by the nick. Upon incorporation of the antisense strand into RISC, the sense strand is separated into fragments at the position of the nick.

The gene expression control mechanism by miRNA typically refers to sequence-specific degradation or translational suppression of RNA induced by miRNA. Mature miRNA is incorporated into miRNA-induced silencing complex (miRISC) containing Argonaute (AGO) protein so that the mature miRNA mainly suppresses translation from mRNA by inducing the miRISC to the mRNA complementary to a seed sequence (sequence from positions 2 to 8 from the 5' end), and also promotes the degradation of the mRNA by degrading the 3'-terminal poly-A tail of the mRNA for truncation.

The gene expression control mechanism by piRNA typically refers to sequence-specific degradation of RNA induced by piRNA. piRNA is incorporated into piRNA-induced silencing complex (piRISC) containing Piwi protein, a protein of the PIWI subfamily, and then translocated into the nucleus to suppress the transcription of the target gene in a sequence-specific manner. rasiRNA (repeat associated siRNA) also induce gene silencing via Piwi protein.

The microRNA mimic is a nucleic acid molecule that mimics the function of endogenous microRNA, and is well known in the art (e.g., van Rooij and Kauppinen, EMBO Mol Med. 2014; 6 (7): 851-64; and Chorn et al., RNA. 2012; 18 (10): 1796-804). The microRNA mimic may contain a chemical modification, and its nucleotide sequence may be changed with respect to the endogenous microRNA. The chemical modification may include various modifications mentioned later. Non-limiting examples of the chemical modification preferably include 2'-fluoro modification, 2'-O-methyl modification, 2'-O-methoxyethyl modification, LNA, phosphorothioate bonds, morpholino, and PNA. The microRNA mimic may have a nucleotide sequence identical to that of the endogenous microRNA (e.g., pre-microRNA) or may have a nucleotide sequence having a deletion, substitution, or addition of one or more nucleotides in the nucleotide sequence of the endogenous microRNA. The microRNA mimic may be double-stranded or single-stranded. The double-stranded one may have one or more single-stranded portions.

shRNA is a single-stranded RNA molecule having an antisense region and a sense region having complementarity to each other, and a loop region interposed therebetween, and assumes a hairpin-shaped three-dimensional structure formed from a duplex region by the pairing of the antisense region and the sense region. shRNA is cleaved by dicer in cells to form a double-stranded siRNA molecule, which is in turn incorporated into RISC, causing RNA interference. shRNA is typically expressed in cells through a nucleic acid construct encoding this shRNA, a plasmid comprising the nucleic acid construct, or the like, and exerts its effect. The shRNA molecule may be delivered directly to a cell.

The inhibitory nucleic acid can be prepared on the basis of information on the gene sequence, etc., of Hic-5 (also called transforming growth factor beta-1-induced transcript 1 protein (TGFB1I1), androgen receptor coactivator 55 kDa protein, androgen receptor-associated protein of 55 kDa, or ARA55). For example, the gene sequence of human Hic-5 is registered under accession number NM_001042454 (transcript variant 1), NM_015927 (transcript variant 2), NM_001164719 (transcript variant 3), and the sequences are as shown in SEQ ID NOs: 1, 3, and 5, respectively (corresponding amino acid sequences are shown in SEQ ID NOs: 2, 4, and 6). For example, the following is known as the gene sequence of nonhuman animal Hic-5. Dog: accession number XM_022419832, XM_005621231, XM_014114355 and XM_005621229. Cat: accession number XM_019820649, XM_019820647, XM_003998629, XM_006942108 and XM_019820648. Horse: accession number XM_023655571 and XM_023655570. Cow: accession number BC104510. Mouse: accession number NM_001289550. siRNA design methods are described, e.g., in Naito and Ui-Tei, Front Genet. 2012;3:102, and miRNA design methods are described, e.g., in Mickiewicz et al., Acta Biochim Pol. 2016;63(1):71-77.

As a sequence of siRNA against Hic-5, e.g., the following is known :5'-UCUGUGAGCUAGACCGUUU-3' (mouse, SEQ ID NO:7), 5'-GGGAAUGCCUUGCGCCCCUU-3' (mouse, SEQ ID NO:8), 5'-AGUGCUACULTCTGAGCGCLTCT-3' (mouse, SEQ ID NO:9), 5'-GGGACAAGGAUCAUCUAUA-3' (mouse, SEQ ID NO:10) (Petropoulos et al., J Cell Biol. 2016;213(5):585-99), 5'-CCUUGCAALTCTCCAGCGAAU-3' (human, SEQ ID NO:11) (Qian et al., Biomed Pharmacother. 2020;121:109355), 5'-GGAGCUGGAUAGACUGAUG-3' (human, SEQ ID NO:12), 5'-CAUCAGUCUAUCCAGCUCC-3' (human, SEQ ID NO:13), 5'-GGACCAGUCUGAAGAUAAG-3' (human, SEQ ID NO:14) (Deakin et al., Mol Biol Cell. 2011;22(3):327-41), 5'-GGAGCUGGAUAGACUGAUGUU-3' (human, SEQ ID NO:15), 5'-GGACCAGUCUGAAGAUAAGLTCT-3' (human, SEQ ID NO:16) (Desai et al., J Biol Chem. 2014;289(26):18270-8), 5'-GCAGCAGCTTCTTCGAGAA-3' (human, SEQ ID NO:17) (Du et al., Cell Death Dis. 2019;10(12):873). As shRNA against Hic-5, e.g., the following is known: the one targeting 5'-GCAGCAGCTTCTTCGAGAA-3' (human, SEQ ID NO:17) (Du et al., supra), the one targeting 5'-TCTCTGACTTCCGCGTTCAAA-3 ' (human, SEQ ID NO:18), or 5'-TCAGTTCAACATCACAGATGA-3' (human, SEQ ID NO:19) (Mori et al., FEBS J. 2019;286(3):459-478). Further examples of the sequence of siRNA against Hic-5 include 5'-CCACUUGCCCAGCUAUAGG-3' (mouse, SEQ ID NO:26) and 5'-UGUGCUGUAUAGAUGAUCC-3' (rat, SEQ ID NO:27).

The inhibitory nucleic acid of the present disclosure may comprise an unmodified nucleotide and/or a modified nucleotide. In the present specification, the unmodified nucleotide and the modified nucleotide are simply referred to as a "nucleotide" collectively. The unmodified nucleotide refers to a naturally occurring nucleotide constituting DNA or RNA, i.e., the one constituted by a nucleobase (adenine, guanine, uracil, thymine, or cytosine), a sugar (ribose or deoxyribose), and a phosphate group. In an unmodified nucleic acid molecule constituted by unmodified nucleotides, the 3' position of one of two adjacent unmodified nucleotides is usually linked to the 5' position of the other unmodified nucleotide through a phosphodiester bond. In one embodiment, the unmodified nucleotide is an unmodified ribonucleotide, and the unmodified nucleic acid molecule is constituted by unmodified ribonucleotides.

The modified nucleotide refers to a nucleotide containing a chemical modification to the unmodified nucleotide. The modified nucleotide may be artificially synthesized or may occur naturally. The modified nucleotide includes a nucleotide modified at its nucleobase, sugar, backbone (internucleotide bond), 5' end and/or 3' end. The modified nucleotide also includes a nucleotide modified at any one of these sites as well as a nucleotide modified at two or more of the sites.

Examples of the modification to the nucleobase include, but are not limited to, 2,4-difluorotoluyl, 2,6-diamino, 5-bromo, 5-iodo, 2-thio, dihydro, 5-propynyl, and 5-methyl modifications, and elimination of a base. Examples of modified nucleobase include, but are not limited to, xanthine, hypoxanthine, inosine, 2-aminoadenine, 6-methyl and other alkyl derivatives of adenine and guanine, universal base, 2-propyl and other alkyl derivatives of adenine and guanine, 5-halouracil and 5-halocytosine, 5-propynyl uracil and 5-propynyl cytosine, 6-azo uracil, 6-azo cytosine and 6-azo thymine, 5-uracil (pseudouracil), 4-thiouracil, 8-halo, amino, thiol, thioalkyl, hydroxyl and other 8-substituted adenines and guanines, 5-trifluoromethyl and other 5-substituted uracils and cytosines, 7-methylguanine, acyclonucleotides, deazapurines, heterocyclic substituted analogs of purines and pyrimidines, e.g., aminoethyoxy phenoxazine, derivatives of purines and pyrimidines (e.g., 1-alkyl-, 1-alkenyl-, heteroaromatic- and 1-alkynyl derivatives) and tautomers thereof, 8-oxo-N6-methyladenine, 7-diazaxanthine, 5-methylcytosine, 5-methyluracil, 5-(1-propynyl) uracil, 5-(1-propynyl) cytosine and 4,4-ethanocytosine, non-purinyl and non-pyrimidinyl bases such as 2-aminopyridine and triazines, abasic nucleotide, deoxy abasic nucleotide, inverted abasic nucleotide, inverted deoxy abasic nucleotide, and the like.

Examples of the modification to the sugar include, but are not limited to: modifications at position 2', e.g., 2'-O-alkyl modifications (e.g., 2'-O-methyl modification and 2'-O-ethyl modification), 2'-methoxyethoxy modification, 2'-methoxyethyl modification, 2'-deoxy modification, 2'-halogen modifications (2'-fluoro modification, 2'-chloro modification, 2'-bromo modification, etc.), 2'-O-allyl modification, 2'-amino modification, 2'-S-alkyl modification, 2'-O-[2(methylamino)-2-oxoethyl] modification, 2'-alkoxy modification, 2'-O-2-methoxyethyl, 2'-allyloxy (-OCH₂CH=CH₂), 2'-propargyl, 2'-propyl, 2'-O-(N-methyl carbamate) modification, 2'-O-(2,4-dinitrophenyl) modification, and 2'-deoxy-2'-fluoro-β-D-arabino modification; modifications at position 4', e.g., 4'-thio modification and 4'-C-hydroxymethyl modification; and other modifications with ethynyl, ethenyl, propenyl, CF, cyano, imidazole, carboxylate, thioate, C₁ to C₁₀ lower alkyl, substituted lower alkyl, alkaryl or aralkyl, OCF₃, OCN, O-, S- or N-alkyl, O-, S- or N-alkenyl, SOCH₃, SO₂CH₃, ONO₂, NO₂, N₃, heterocycloalkyl, heterocycloalkaryl, aminoalkylamino, polyalkylamino or substituted silyl. Other examples of the modified sugar include locked nucleic acid (LNA), oxetane-LNA (OXE), unlocked nucleic acid (UNA), ethylene-bridged nucleic acid (ENA), altriol nucleic acid (ANA), and hexitol nucleic acid (HNA).

In the present disclosure, alkyl group includes saturated aliphatic groups, including straight-chain alkyl groups (e.g., methyl, ethyl, propyl, butyl, pentyl, hexyl, heptyl, octyl, nonyl, decyl, etc.), branched-chain alkyl groups (isopropyl, tert-butyl, isobutyl, etc.), cycloalkyl (alicyclic) groups (cyclopropyl, cyclopentyl, cyclohexyl, cycloheptyl, cyclooctyl), and alkyl substituted cycloalkyl groups. In certain embodiments, a straight chain or branched chain alkyl has 6 or fewer carbon atoms in its backbone (e.g., C₁-C₆ for straight chain, C₃-C₆ for branched chain), and more preferably 4 or fewer. Likewise, preferred cycloalkyls may have from 3-8 carbon atoms in their ring structure, and more preferably have 5 or 6 carbons in the ring structure. The term C₁-C₆ includes alkyl groups containing 1 to 6 carbon atoms. The alkyl group can be substituted alkyl group such as alkyl moieties having substituents replacing a hydrogen on one or more carbons of the hydrocarbon backbone. Such substituents can include, e.g., alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

In the present disclosure, alkoxy group includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, etc.

In the present disclosure, halogens include fluorine, bromine, chlorine, iodine.

Examples of modified backbone include, but are not limited to phosphorothioate, thiophosphate-D-ribose entities, triester, thioate, 2'-5' bridged backbone (may also be referred to as 5'-2' or 2'S' nucleotide or 2'S' ribonucleotide), PACE, 3'-(or -5')deoxy-3'-(or -5')thio-phosphorothioate, phosphorodithioate, phosphoroselenates, 3'-(or -5')deoxy phosphinates, borano phosphates, 3'-(or - 5')deoxy-3'-(or 5'-)amino phosphoramidates, hydrogen phosphonates, phosphonates, borano phosphate esters, phosphoramidates, alkyl or aryl phosphonates and phosphotriester modifications such as alkylphosphotriesters, phosphotriester phosphorus linkages, 5'-ethoxyphosphodiester, P-alkyloxyphosphotriester, methylphosphonate and morpholino, and nonphosphorus containing linkages e.g., carbonate, carbamate, silyl, sulfur, sulfonate, sulfonamide, formacetal, thioformacetyl, oxime, methyleneimino, methylenemethylimino, methylenehydrazo, methylenedimethylhydrazo and methyleneoxymethylimino linkages.

Examples of 5'- and/or 3'- end modification include addition of a capping moiety to 5'- and/or 3'- end, and modification at terminal phosphate groups, such as [3'-3']-inverted deoxyribose, deoxyribonucleotide, [5'-3']-3'-deoxyribonucleotide, [5'-3']-ribonucleotide, [5'-3']-3'-O-methyl ribonucleotide, 3'-glyceryl, [3'-5']-3'-deoxyribonucleotide, [3'-3']-deoxyribonucleotide, [5'-2']-deoxyribonucleotide, and [5'-3']-dideoxyribonucleotide. Non-limiting examples of capping moiety include an abasic nucleotide, a deoxy abasic nucleotide, an inverted (deoxy) abasic nucleotide, a hydrocarbon (alkyl) moiety and derivatives thereof, a mirror nucleotide (L-DNA or L-RNA), bridged nucleic acids including LNA and ethylene bridged nucleic acids, linkage modified nucleotides (e.g. PACE) and base modified nucleotides, glyceryl, dinucleotide, acyclic nucleotide, amino, fluoro, chloro, bromo, CN, CF, methoxy, imidazole, carboxylate, thioate, C₁ to C₁₀ lower alkyl, substituted lower alkyl, alkaryl or aralkyl, OCF₃, OCN, O , S , or N-alkyl, O , S , or N-alkenyl, SOCH₃, SO₂CH₃, ONO₂, NO₂, N₃, heterocycloalkyl, heterocycloalkaryl, aminoalkylamino, polyalkylamino or substituted silyl. The capping moiety may serve as a non-nucleotide overhang.

Modified nucleotides of the present disclosure include 2'-deoxyribonucleotides, 2'-O-methyl ribonucleotides, 2'-deoxy-2'-fluoro ribonucleotides, universal base nucleotides, acyclic nucleotides, 5-C-methyl nucleotides, nucleotides containing biotin group, and terminal glyceryl and/or inverted deoxy abasic residue, nucleotide containing sterically hindered molecules, such as fluorescent molecules and the like, 3'-deoxyadenosine (cordycepin), 3'-azido-3'-deoxythymidine (AZT), 2',3'-dideoxyinosine (ddI), 2',3'-dideoxy-3'-thiacytidine (3TC), 2',3'-didehydro-2',3'-dideoxythymidine (d4T), nucleotides containing 3'-azido-3'-deoxythymidine (AZT), 2',3'-dideoxy-3'-thiacytidine (3TC) or 2',3'-didehydro-2',3'-dide-oxythymidine (d4T), a nucleotide having a Northern conformation, 2'-methyl-thio-ethyl, 2'-deoxy-2'-fluoro nucleotides, 2'-deoxy-2'-chloro nucleotides, 2'-azido nucleotides, and 2'-O-methyl nucleotides, 6-membered ring nucleotide analogs including hexitol and altritol nucleotide monomers disclosed in WO 2006/047842, etc., mirror nucleotides (e.g., L-DNA (L-deoxyriboadenosine-3'-phosphate (mirror dA), L-deoxyribocytidine-3'-phosphate (mirror dC), L-deoxyriboguanosine-3'-phosphate (mirror dG), L-deoxyribothymidine-3'-phosphate (mirror image dT)) and L-RNA (L-riboadenosine-3'-phosphate (mirror rA), L-ribocytidine-3'-phosphate (mirror rC), L-riboguanosine-3'-phosphate (mirror rG), L-ribouracil-3'-phosphate (mirror dU), etc.).

Non-limiting examples of the modified nucleotide are also described, e.g., in Gaglione and Messere, Mini Rev Med Chem. 2010; 10 (7): 578-95, Deleavey and Damha, Chem Biol. 2012; 19 (8): 937-54, and Bramsen and Kjems, J. Front Genet. 2012; 3: 154.

The genome editing molecule is a molecule or a molecule set capable of modifying a genome sequence in a site-specific manner. Examples thereof include, but are not limited to, molecules based on CRISPR/Cas system, TALEN, ZFN, or meganuclease (MN). The genome editing molecule based on CRISPR/Cas system functions as a set of guide RNA and Cas (CRISPR-associated protein). Examples of Cas contained in CRISPR/Cas system include, but are not limited to, Cas9, Cas12a(Cpf1), Cas12b, Cas13, xCas9, VQR, VRER, spCas9-NG, spCas9-HF1, Cas nickase (e.g., Cas9 nickase), eSpCas9, evoCas9, HypaCas9, CjCas9, Split-Cas (e.g., Split-Cas9), dCas-BE (e.g., dCas9-BE), and the like (Breeders et al., iScience. 2019;23(1):100789). CRISPR/Cas13 can edit RNA and as such, can be used in gene silencing similar to that of RNAi.

In the present disclosure, the antibody includes a whole antibody and an antigen binding fragment thereof. The class of antibody is not particularly limited and includes IgA, IgD, IgE, IgG and IgM, including IgA1, IgA2, IgG1, IgG2a, IgG2b, IgG3 and IgG4 as subclasses. The whole antibody also includes IgNAR and heavy chain antibodies. The antigen binding fragment includes Fab, Fab', F(ab')₂, Fd, Fcab, vNAR and VHH (nanobody). The antibody against the antigen Hic-5 (anti-Hic-5 antibody) is commercially available or can be obtained by a well-known approach, e.g., by immunizing animals, e.g., mice, rats, rabbits, goats, or sheep, with Hic-5 or its immunogenic fragment, and isolating serum, or by isolating anti-Hic-5 antibody-producing cells from such immunized animals, and culturing the cells, if necessary, after immortalization. The antibody may be polyclonal or monoclonal and may be a chimeric antibody, a humanized antibody or a human antibody. Hic-5 serving as an antigen is preferably human Hic-5. Hic-5 of another animal species may be used as the antigen as long as the resulting antibody is capable of recognizing human Hic-5. In another embodiment, the antibody may be an equine antibody that recognizes equine Hic-5, a canine antibody that recognizes canine Hic-5, a feline antibody that recognizes feline Hic-5, or a bovine antibody that recognizes bovine Hic-5.

The antigen binding derivative of the antibody includes an antibody derivative having antigen binding moieties (e.g., VH region, VL region, and Fv region) of the antibody combined with other moieties (e.g., CH1 region, CH2 region, CH3 region, CL region, and Fc region) of the antibody, e.g., scFv, minibody, scFv-Fc, scFv₂ (diabody), scFvs (triabody), scFv₄ (tetrabody), Fv-clasp (Arimori et al., Structure. 2017;25(10):1611-1622), BIf (bispecific scFv immunofusion, Kuo et al., Protein Eng Des Sel. 2012;25(10):561-9), and a bispecific antibody. Examples of the Hic-5 binding antibody mimetic include monobody (adnectin), affibody, Affimer, affitin, Anticalin, atrimer, finomer, armadillo repeat protein, Kunitz domain, nottin, avimer, DARPin, alphabody, O body, and repebody (Simeon and Chen, Protein Cell. 2018;9(1):3-14, Yu et al., Annu Rev Anal Chem (Palo Alto Calif). 2017;10(1):293-320, Wuo and Arora, Curr Opin Chem Biol. 2018 Jun;44:16-22).

When the Hic-5 inhibitor is a polypeptide (polypeptide-type Hic-5 inhibitor, e.g., an antibody, an antigen binding derivative of the antibody, an antibody mimetic, or a dominant negative mutant), the composition A of the present disclosure may comprise a nucleic acid molecule encoding the polypeptide-type Hic-5 inhibitor instead of this inhibitor. Such a nucleic acid molecule is also included in the Hic-5 inhibitor of the present disclosure. The nucleic acid molecule encoding the polypeptide-type Hic-5 inhibitor is expressed in cells so that the Hic-5 inhibitor acts in the cells and can thereby produce the desired effect. Use of the nucleic acid molecule encoding the polypeptide-type Hic-5 inhibitor is particularly useful, e.g., when the transfer of the nucleic acid molecule into cells is easier than that of the polypeptide.

In the present disclosure, the cardiac hypertrophy refers to a thickened state of the cardiac wall and includes concentric cardiac hypertrophy and eccentric cardiac hypertrophy. The cardiac hypertrophy is caused by hypertension, cardiac valvular disease, septal defect, hypertrophic cardiomyopathy, or the like. The cardiac hypertrophy may be a compensated cardiac hypertrophy or a decompensated cardiac hypertrophy. The cardiac hypertrophy may be associated with heart enlargement. The heart enlargement refers to a state having a cardiothoracic ratio of 50% or more. In one embodiment, the cardiac hypertrophy according to the present disclosure is caused by an increased left ventricular load. The increased left ventricular load may occur due to hypertension, hypertrophic cardiomyopathy, aortic valvular stenosis, or the like. Thus, the composition of the present disclosure for use in the treatment of cardiac hypertrophy may be applied to a subject having hypertension, hypertrophic cardiomyopathy and/or aortic valvular stenosis. In one embodiment, the cardiac hypertrophy according to the present disclosure is a compensated cardiac hypertrophy. The composition of the present disclosure for use in the treatment of cardiac hypertrophy can be applied to a subject diagnosed with cardiac hypertrophy or a subject having a risk of developing cardiac hypertrophy.

In the present disclosure, the interstitial pneumonia refers to a pathological condition in which the alveolar wall has become fibrotic due to inflammation or damage in the alveolar wall, leading to impaired gas exchange. The interstitial pneumonia includes infection-induced interstitial pneumonia associated with viral pneumonia, fungal pneumonia, or mycoplasmal pneumonia, interstitial pneumonia associated with collagenosis such as rheumatoid arthritis, systemic scleroderma, dermatomyositis, polymyositis, or mixed connective tissue disease (MCTD), interstitial pneumonia associated with radiation exposure, drug-induced interstitial pneumonia ascribable to an anticancer agent such as bleomycin, a Chinese herb such as shosaikoto, interferon, an antibiotic, or paraquat, pulmonary fibrosis caused by such interstitial pneumonia, and idiopathic interstitial pneumonia (IIP). The idiopathic interstitial pneumonia includes chronic fibrotic interstitial pneumonia including idiopathic pulmonary fibrosis (IPF) and nonspecific interstitial pneumonia (NSIP), smoking-related interstitial pneumonia (SRIF) including respiratory bronchiolitis-associated interstitial lung disease (RB-ILD) and desquamative interstitial pneumonia (DIP), acute/subacute interstitial pneumonia including cryptogenic organizing pneumonia (COP) and acute interstitial pneumonia (AIP), lymphocytic interstitial pneumonia (LIP), pleuroparenchymal fibroelastosis (PPFE), and unclassified interstitial pneumonia. In one embodiment, the interstitial pneumonia includes idiopathic interstitial pneumonia, particularly, idiopathic pulmonary fibrosis. The composition of the present disclosure for use in the treatment of interstitial pneumonia can be applied to a subject diagnosed with interstitial pneumonia or a subject having a risk of developing interstitial pneumonia.

In the present disclosure, the osteoarthritis (OA) refers to chronic arthrosis characterized by destruction or loss of articular cartilages. Its pathological condition may be associated with osteophyte formation, narrowing of joint cleft, subchondral bone sclerosis, disappearance of joint cleft, or the like. The osteoarthritis includes primary (idiopathic) and secondary ones. The secondary osteoarthritis may be caused by a pathological condition that changes cartilage microenvironments, e.g., major trauma, congenital joint abnormality, metabolism disorder (hemochromatosis, Wilson's disease, etc.), infection, endocrine disease, neuropathic disease, or a disease that changes the normal structure and function of hyaline cartilages (rheumatoid arthritis, gout, chondrocalcinosis, etc.). The osteoarthritis treatment composition of the present disclosure can be applied to a subject diagnosed with osteoarthritis or a subject having a risk of developing osteoarthritis.

In the present disclosure, the fatty liver refers to a pathological condition characterized by fat accumulation in the liver and includes alcoholic fatty liver and non-alcoholic fatty liver disease (NAFLD). NAFLD further includes NASH (non-alcoholic steatohepatitis) which tends to progress to liver cirrhosis or liver cancer, and non-alcoholic fatty liver (NAFL) which often has a relatively benign course. NASH is steatohepatitis characterized by hepatocellular injury, inflammation, hepatocellular ballooning and/or fibrosis. NASH includes liver cirrhosis ascribable to the progression of NASH, and liver cancer ascribable to the progression of NASH. The treatment of NASH leads to the treatment of liver cirrhosis or liver cancer ascribable to the progression of NASH, particularly, the prevention of these diseases. The composition of the present disclosure for use in the treatment of fatty liver can be applied to a subject diagnosed with fatty liver or a subject having a risk of developing fatty liver. In some embodiments, the composition A4 of the present disclosure is used for treatment of NASH. In some embodiments, the composition A4 of the present disclosure is used for reducing fat accumulated in the liver. In a particular embodiment, the composition A4 of the present disclosure is used for reducing fat accumulated in the liver having NASH.

In the present disclosure, the tumor includes benign tumor and malignant tumor (cancer). The cancer includes epithelial malignant tumor and non-epithelial malignant tumor. A tumor for the composition A of the present disclosure includes solid tumor. Solid tumor includes, but not limited to, e.g., brain tumor, head and neck tumor, breast tumor, lung tumor, esophageal tumor, thyroid tumor, stomach tumor, small intestine tumor, appendix tumor, colorectal tumor, rectal tumor, liver tumor, pancreatic tumor, gallbladder tumor, bile duct tumor, anal tumor, kidney tumor, renal pelvis and ureter tumor, bladder tumor, prostate tumor, penile tumor, testicular tumor, uterine tumor, ovarian tumor, vulvar tumor, vaginal tumor, skin tumor, fibrosarcoma, fibrous histiocytoma, lipoma, liposarcoma, rhabdomyoma, rhabdomyosarcoma, leiomyoma, leiomyosarcoma, angioma, angiosarcoma, Kaposi's sarcoma, lymphangioma, lymphangiosarcoma, synovialoma, synovial sarcoma, chondroma, chondrosarcoma, osteoma, osteosarcoma, myeloma, lymphoma and GIST. In a particular embodiment, the tumor is colorectal tumor. The tumor may be present in any site, e.g., the brain, the head and the neck, the chest, extremities, the lung, the heart, thymus gland, the esophagus, the stomach, the small intestine (the duodenum, the jejunum, and the ileum), the large intestine (the colon, the cecum, the appendix, and the rectum), the liver, the pancreas, the gallbladder, the anus, the kidney, the ureter, the bladder, the prostate, the penis, the testis, the uterus, the ovary, the vulva, the vagina, the skin, striated muscle, smooth muscle, synovium, cartilage, bone, thyroid gland, adrenal gland, peritoneum, mesentery, bone marrow, the vascular system, and the lymphatic system such as lymph nodes.

In some embodiments, the composition A of the present disclosure is used in the treatment of a cancer. The cancer includes solid cancer. Solid cancer includes, but not limited to, e.g., brain tumor, head and neck cancer, breast cancer, lung cancer, esophageal cancer, thyroid cancer, stomach cancer, small intestine cancer, appendix cancer, colorectal cancer (CRC), rectal cancer, liver cancer, pancreatic cancer, gallbladder cancer, bile duct cancer, anal cancer, kidney cancer, renal pelvis and ureter cancer, bladder cancer, prostate cancer, penile cancer, testicular cancer, uterine cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and GIST. The cancer may be present in any site, e.g., the brain, the head and the neck, the chest, extremities, the lung, the heart, thymus gland, the esophagus, the stomach, the small intestine (the duodenum, the jejunum, and the ileum), the large intestine (the colon, the cecum, the appendix, and the rectum), the liver, the pancreas, the gallbladder, the anus, the kidney, the ureter, the bladder, the prostate, the penis, the testis, the uterus, the ovary, the vulva, the vagina, the skin, striated muscle, smooth muscle, synovium, cartilage, bone, thyroid gland, adrenal gland, peritoneum, mesentery, bone marrow, the vascular system, and the lymphatic system such as lymph nodes. The cancer may be accompanied by cancer-associated fibroblasts (CAFs). In some embodiments, the cancer is primary cancer. In another embodiment, the cancer is metastatic cancer. In a particular embodiment, the cancer is colorectal cancer, particularly, primary colorectal cancer. The colorectal cancer includes adenocarcinoma, squamous cell cancer, and adenosquamous cancer. Colorectal adenocarcinoma includes papillary adenocarcinoma, tubular adenocarcinoma, poorly differentiated adenocarcinoma, mucinous cancer, signet ring cell adenocarcinoma, and medullary cancer.

In one embodiment, the tumor (including cancer) to be treated with the composition A of the present disclosure is an established (i.e., pre-existing) tumor. In a preferred embodiment, the composition A5 of the present disclosure not only suppresses tumor growth but also reduces tumor size. In another preferred embodiment, the composition A5 of the present disclosure has no adverse effect or has minor influence, if any, on normal tissues. In some embodiments, the composition A5 of the present disclosure suppresses the vascularization of tumor tissues and/or eliminates blood vessels present in tumor tissues.

In another embodiment, the present disclosure provides a composition for suppressing the vascular invasion of cancer cells, comprising a Hic-5 inhibitor (hereinafter, may be referred to as the "composition of the present disclosure for use in suppressing vascular invasion" or the "composition B of the present disclosure").

The Hic-5 inhibitor in the composition B of the present disclosure is the same as the one described with respect to the composition A of the present disclosure. The vascular invasion of cancer cells includes invasion into blood vessels and invasion into lymphatic vessels. The cancer cells are not particularly limited as long as the cancer cells are capable of causing vascular invasion. The cancer cells include epithelial malignant tumor cells and non-epithelial malignant tumor cells. A cancer cell for the composition B of the present disclosure includes, but not limited to, e.g., a cell of brain tumor, head and neck cancer, breast cancer, lung cancer, esophageal cancer, thyroid cancer, stomach cancer, small intestine cancer, appendix cancer, colorectal cancer, rectal cancer, liver cancer, pancreatic cancer, gallbladder cancer, bile duct cancer, anal cancer, kidney cancer, renal pelvis and ureter cancer, bladder cancer, prostate cancer, penile cancer, testicular cancer, uterine cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma, GIST and leukemia. The cancer cell may be present in any site, e.g., in the brain, the head and the neck, the chest, extremities, the lung, the heart, thymus gland, the esophagus, the stomach, the small intestine (the duodenum, the jejunum, and the ileum), the large intestine (the colon, the cecum, the appendix, and the rectum), the liver, the pancreas, the gallbladder, the anus, the kidney, the ureter, the bladder, the prostate, the penis, the testis, the uterus, the ovary, the vulva, the vagina, the skin, striated muscle, smooth muscle, synovium, cartilage, bone, thyroid gland, adrenal gland, peritoneum, mesentery, bone marrow, the vascular system, the lymphatic system such as lymph nodes, and lymph.

Suppressing the vascular invasion of cancer cells means reducing the vascular invasion of cancer cells as compared with the case where the Hic-5 inhibitor is not allowed to act. The degree of suppression may be, e.g., a decrease of 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 100% in the number of cancer cells that have caused vascular invasion as compared with the case where the Hic-5 inhibitor is not allowed to act. A greater degree of suppression is more preferred. The number of cancer cells that have caused vascular invasion can be counted by, e.g., histopathological examination.

Since Hic-5 usually resides within cells, it is preferred that the Hic-5 inhibitor in the compositions A and B of the present disclosure is to be delivered into the cells. The delivery of the Hic-5 inhibitor into cells can be performed using, e.g., but not limited to, electroporation, a calcium phosphate method, a dextran method, microinjection, iTOP (D'Astolfo et al., Cell. 2015;161(3):674-690) a cell penetrating peptide (CPP), a viral vector or a nonviral vector (see, e.g., Bruce and McNaughton, Cell Chem Biol. 2017;24(8):924-934, Lee et al., J Control Release. 2019;313:80-95). For in vivo delivery, a delivery approach using a cell penetrating peptide, a viral vector or a nonviral vector is preferred.

The cell penetrating peptide is a peptide that can penetrate a cell membrane and migrate into the cell. Many types thereof are known. Non-limiting examples of the cell penetrating peptide include, TAT, polyarginine (R5, R8, R9, etc.), Penetratin, Pep-1, proline-rich peptide (Pro), TAT-HA2, Hph-1, HP4, LAH4, LAH4-L1, Vectofusin-1, low-molecular-weight protamine (LMWP), LL-37, Pep-7, Pept1, Pept2, IVV-14, Transportant, Ig(v), pVEC, HRSV, TGN, Derived Ku-70, RW(n), RRRRRRGGRRRRG, SVS-1, L-CPP, RLW, K16ApoE, Angiopep-2, ACPP, KAFAK, hCT(9-32), VP22, and the like (see, e.g., Vanova et al., Materials (Basel). 2019;12(17). pii: E2671, Silva et al., Biomolecules. 2019;9(1). pii: E22, Derakhshankhah and Jafari, Biomed Pharmacother. 2018;108:1090-1096). The cell penetrating peptide may be linked directly to the Hic-5 inhibitor (Tai, Molecules. 2019;24(12). pii: E2211), or may be used by linking it to a viral vector or a nonviral vector containing the Hic-5 inhibitor (Vanova et al., supra, Silva et al., supra)

Examples of the viral vector include, e.g., but are not limited to, vectors based on adenovirus, adeno-associated virus (AAV), retrovirus, vaccinia virus, poxvirus, lentivirus, and herpes virus. Examples of the non-viral vector include, but are not limited to, carriers in a particle form such as polymer particles, lipid particles and inorganic particles, and a bacterial vector. Nanoparticles having a nano level of size can be used as the carrier in a particle form. Examples of the polymer particles include, but are not limited to, those comprising polymers such as cationic polymers, polyamidoamine (PAMAM), chitosan, glycol chitosan, cyclodextrin, poly(lactic-co-glycolic acid) (PLGA), poly(lactic-co-caprolactonic acid) (PLCA), poly(β amino ester), atelocollagen and polyethyleneimine (PEI). The lipid particles include liposomes, non-liposomal lipid particles, and the like. The liposome is a vesicle having a lumen surrounded by a lipid bilayer, and the non-liposomal lipid particles are lipid particles having no such structure. Examples of the inorganic particles include gold nanoparticles, quantum dots, silica nanoparticles, iron oxide nanoparticles (e.g., superparamagnetic iron oxide nanoparticles (SPION)), nanotubes (e.g., carbon nanotubes (CNT)), nanodiamond, and fullerene. Examples of the bacterial vector include, but are not limited to, vectors based on Listeria bacterium, bifidus, and salmonella. The nonviral vector can be used in the delivery of not only a nucleic acid molecule but also a polypeptide such as an antibody or Cas. Various modifications can be added to the particle carrier described above. Examples thereof include stealthing with PEG, targeting with a targeting ligand, and modification with a cell penetrating peptide (CPP).

Non-limiting examples of the target cells of the Hic-5 inhibitor include cardiac fibroblasts (including myofibroblasts) and cardiomyocytes for cardiac hypertrophy, pulmonary fibroblasts (including myofibroblasts) and Clara cells for interstitial pneumonia, hepatic stellate cells, myofibroblasts, portal fibroblasts, and bone marrow-derived fibrocytes for fatty liver, chondrocytes for osteoarthritis, tumor cells, fibroblasts in tumor tissues, and CAFs for tumor, and cancer cells for the vascular invasion of cancer cells. For example, the administration of adenovirus vector, retrovirus vector, herpes simplex virus vector, lentivirus vector, AAV vector and various nonviral vectors by intra-articular injection is reported as being effective for in vivo nucleic acid delivery to cartilage tissues (Evans et al., Hum Gene Ther. 2018;29(1):2-14). Also, it has been reported that hyaluronic acid-coated nanoparticles targeting CD44 expressed in chondrocytes were used in drug delivery to chondrocytes (Ansboro et al., Eur Cell Mater. 2012;23:310-8). Thus, hyaluronic acid can be used as a targeting ligand for chondrocytes.

The Hic-5 inhibitor may be conjugated with a functional moiety such as the polymer, lipid, targeting ligand, or CPP described above by binding. Such a conjugate permits formulation of the Hic-5 inhibitor without using the carrier described above.

In one embodiment, the Hic-5 inhibitor contained in the compositions A and B of the present disclosure exists as a conjugate with a functional moiety that assists in delivery to cells, e.g., a polymer, a lipid, a targeting ligand, or CPP. In another embodiment, the Hic-5 inhibitor contained in the compositions A and B of the present disclosure is incorporated in a viral vector or a nonviral vector, or exists in a state bound with such a vector.

In some embodiments, the Hic-5 inhibitor contained in the compositions A and B of the present disclosure is not allowed to target particular cells. Since Hic-5 KO mice are normally born and grow (Kim-Kaneyama et al., J Atheroscler Thromb. 2012;19(7):601-7), systemic suppression of Hic-5 is considered to have no adverse effect on the normal function of the living body. On the other hand, the development or progression of cardiac hypertrophy, interstitial pneumonia, osteoarthritis, fatty liver, tumor and the vascular invasion of cancer cells revealed this time as well as other diseases involving Hic-5, such as arteriosclerosis, is suppressed in the Hic-5 KO mice. Therefore, the Hic-5 inhibitor is administered to a subject, without targeting particular cells, and is thereby not only capable of concurrently treating a plurality of diseases involving Hic-5 which are to be treated, but capable of treating a latent disease involving Hic-5 which is not detected in the subject.

The compositions A and B of the present disclosure may be administered through various routes including both oral and parenteral routes, e.g., but not limited to, oral, buccal, mouth, intravenous, intramuscular, subcutaneous, intradermal, local, rectal, intraarterial, intraportal, intraventricular, intramyocardial, intra-articular, transmucosal, transdermal, intranasal, intraperitoneal, intra airway, intrabronchial, intra-alveolar, intrapulmonary, intrahepatic and intrauterine routes, and may be formulated into a dosage form suitable for each administration route. As such a dosage form and a formulation method, any ones known in the art can be appropriately adopted (see e.g., Remington's Pharmaceutical Sciences, 18th Ed., Mack Publishing Co., Easton, PA (1990)).

Examples of the dosage form suitable for oral administration include, but are not limited to, powders, granules, tablets, capsules, solutions, suspensions, emulsions, gels, and syrups. Examples of the dosage form suitable for parenteral administration include injections such as solution-type injections, suspension-type injections, emulsion-type injection, and injections to be prepared at the time of use. The preparation for parenteral administration may be in the form of an aqueous or non-aqueous isotonic sterile solution or suspension. Delivery to lung tissues can be performed using, e.g., the form of an aerosolized agent or a spray-dried formulation suitable for administration with an inhalation apparatus or a nebulizer.

The compositions A and B of the present disclosure may comprise one or more pharmaceutically acceptable additives (e.g., surfactants, carriers, diluents, and excipients). The pharmaceutically acceptable additives are well known in the medical field and described, e.g., in Remington's Pharmaceutical Sciences, 18th Ed., Mack Publishing Co., Easton, PA (1990), which is incorporated herein by reference in its entirety.

In the present disclosure, the "treatment" includes every type of medically acceptable prophylactic and/or therapeutic intervention aimed at cure, transient remission or prevention, etc., of a disease. The "treatment" includes medically acceptable intervention for various purposes including, e.g., the delay or arrest of progression of a disease, the regression or disappearance of a lesion, the prevention of onset of the disease or the prevention of recurrence of the disease.

In the present disclosure, the "subject" means any individual organism, preferably animal, more preferably mammalian, further preferably human individual. In another embodiment, the subject may be a nonhuman animal such as a companion animal or a farm animal, particularly, a dog, a cat, a horse, a donkey, a bovine, a goat, or a sheep. The subject may be healthy (e.g., having no particular disease or not having any disease) or may be affected by some disease. When the treatment, etc., of a disease is intended, the subject typically means a subject affected by the disease or having a risk of being affected by the disease.

Another embodiment of the present disclosure relates to a method of treating a disease selected from cardiac hypertrophy, interstitial pneumonia, osteoarthritis, fatty liver and tumor, the method comprising administering a therapeutically effective amount of a Hic-5 inhibitor or a composition comprising this inhibitor to a subject in need thereof (hereinafter, may be referred to as the "treatment method of the present disclosure"). In this context, the effective amount is, e.g., an amount that prevents the development and recurrence of the disease, or cures the disease. The subject in the present method includes, but is not limited to, a subject diagnosed as having a disease selected from cardiac hypertrophy, interstitial pneumonia, osteoarthritis, fatty liver and tumor, and a subject having a risk of developing the disease. Examples of the subject having a risk of developing the disease include, but are not limited to, subjects having a state causative of the disease. Specific examples thereof include: for cardiac hypertrophy, subjects having hypertension, cardiac valvular disease, septal defect, hypertrophic cardiomyopathy, or the like; for interstitial pneumonia, subjects having pulmonary infection, collagenosis, radiation exposure, a smoking history, a history of exposure to a drug capable of inducing interstitial pneumonia, or the like; for osteoarthritis, elderly and/or obese subjects, and subjects in an environment with overuse of joints; for fatty liver, subjects having obesity, type 2 diabetes mellitus, hypercholesteremia, hypertension, metabolic syndrome, or the like; and for tumor, subjects having radiation exposure, a smoking history, a drinking history, a history of exposure to a carcinogen, or the like. In a particular embodiment, the subject in the present method includes a subject having a disease selected from cardiac hypertrophy, interstitial pneumonia, osteoarthritis, fatty liver and tumor, and detected by the disease detection method of the present disclosure mentioned later. The type of the disease and the Hic-5 inhibitor in the treatment method of the present disclosure are as described above with respect to the composition A of the present disclosure.

Another embodiment of the present disclosure relates to a method for suppressing a vascular invasion of cancer cells, the method comprising administering an effective amount of a Hic-5 inhibitor or a composition comprising this inhibitor to a subject in need thereof (hereinafter, may be referred to as the "vascular invasion suppression method of the present disclosure"). In this context, the effective amount is an amount capable of suppressing the vascular invasion of cancer cells. The subject in the present method typically includes a subject diagnosed as having a cancer. The Hic-5 inhibitor in the vascular invasion suppression method of the present disclosure is as described above with respect to the composition A of the present disclosure. The type of the cancer cells and the suppression of the vascular invasion are as described above with respect to the composition B of the present disclosure.

In the above methods, the specific dose of the Hic-5 inhibitor or the composition to be administered to the subject may be determined in consideration of various conditions as to the subject in need of the administration, e.g., the purpose of the method, a therapeutic regimen, the type of the disease, the severity of symptoms, the general health state, age, and body weight of the subject, the sex of the subject, diets, the timing and frequency of administration, a concurrent drug, responsiveness to therapy, and compliance with therapy. The total daily dose of the Hic-5 inhibitor or the composition is not limited and may be, e.g., about 1 µg/kg to about 1000 mg/kg body weight, about 10 µg/kg to about 100 mg/kg body weight, or about 100 µg/kg to about 10 mg/kg body weight, in terms of the amount of the Hic-5 inhibitor. Alternatively, the dose may be calculated on the basis of the surface area of a patient.

The administration route includes various routes including both oral and parenteral routes, e.g., oral, buccal, mouth, intravenous, intramuscular, subcutaneous, intradermal, local, rectal, intraarterial, intraportal, intraventricular, intramyocardial, intra-articular, transmucosal, transdermal, intranasal, intraperitoneal, intra-airway, intratracheal, intrabronchial, intra-alveolar, intrapulmonary and intrauterine routes.

The frequency of administration differs depending on the properties of the preparation or the composition used or the conditions of the subject as described above and may be, e.g., plural times per day (i.e., 2, 3, 4 or 5 or more times per day), once a day, every few days (i.e., every 2, 3, 4, 5, 6, or 7 days), several times a week (e.g., 2, 3, or 4 times a week), every week, or every few weeks (i.e., every 2, 3, or 4 weeks).

Another embodiment of the present disclosure relates to a Hic-5 inhibitor for use in the treatment of a disease selected from cardiac hypertrophy, interstitial pneumonia, osteoarthritis, fatty liver and tumor. A further embodiment of the present disclosure relates to use of a Hic-5 inhibitor in the treatment of a disease selected from cardiac hypertrophy, interstitial pneumonia, osteoarthritis, fatty liver and tumor. A yet another embodiment of the present disclosure relates to use of a Hic-5 inhibitor in the manufacture of a medicament for treating a disease selected from cardiac hypertrophy, interstitial pneumonia, osteoarthritis, fatty liver and tumor. The type of the disease and the Hic-5 inhibitor in these embodiments are as described above with respect to the composition A of the present disclosure.

Another embodiment of the present disclosure relates to a Hic-5 inhibitor for use in suppressing the vascular invasion of cancer cells. A further embodiment of the present disclosure relates to use of a Hic-5 inhibitor for suppressing the vascular invasion of cancer cells. A yet another embodiment of the present disclosure relates to use of a Hic-5 inhibitor in the manufacture of a medicament for suppressing the vascular invasion of cancer cells. The Hic-5 inhibitor in these embodiments is as described above with respect to the composition A of the present disclosure. The type of the cancer cells and the suppression of the vascular invasion are as described above with respect to the composition B of the present disclosure.

Another embodiment of the present disclosure relates to a method of detecting, diagnosing, testing or assisting the diagnosis of a disease selected from cardiac hypertrophy, interstitial pneumonia, osteoarthritis, NASH and cancer with a risk of vascular invasion, comprising:
detecting the expression of Hic-5 in a tissue suspected of having the disease; and
comparing the degree of expression of Hic-5 in the tissue with the degree of expression of Hic-5 in a normal tissue, wherein
a greater degree of expression of Hic-5 in the tissue suspected of having the disease than the degree of expression of Hic-5t in the normal tissue indicates the presence of the disease (hereinafter, may be referred to as the "disease detecting method of the present disclosure").

Examples of the tissue suspected of having the disease include the cardiac wall, particularly, the myocardium, for cardiac hypertrophy; lung tissues for interstitial pneumonia; articular cartilage tissues, particularly, cartilage tissues of weight-bearing joints of the lower extremity such as knee joints or hip joints, finger joints, and the spine which are favored sites for osteoarthritis; liver tissues for fatty liver; and cancer tissues for cancer. The tissue suspected of having the disease may be derived from a subject suspected of having the disease. Examples of the subject suspected of having the disease include subjects manifesting symptoms related to the disease, and subjects having a condition causative of the disease. Examples thereof include: for cardiac hypertrophy, subjects having hypertension, cardiac valvular disease, septal defect, or hypertrophic cardiomyopathy; for interstitial pneumonia, subjects manifesting respiratory symptoms such as cough or breathlessness, and subjects having pulmonary infection, collagenosis, radiation exposure, a smoking history, or a history of exposure to a drug capable of inducing interstitial pneumonia; for osteoarthritis, subjects manifesting joint pain, elderly and/or obese subjects, and subjects in an environment with overuse of joints; for fatty liver, subjects having obesity, type 2 diabetes mellitus, hypercholesteremia, hypertension, or metabolic syndrome; and for cancer with a risk of vascular invasion, subjects having a cancer. The tissue in which the expression of Hic-5 is to be detected may reside in vivo or may be isolated from the living body.

Examples of the normal tissue include corresponding tissues (e.g., the cardiac wall, lung tissues, articular cartilage tissues, and the same tissues as a cancer-bearing tissue in a test subject) in a subjects who is free of the disease, and normal tissues adjacent to cancer tissues.

The expression of Hic-5 can be detected by various approaches. Approaches for detecting the expression of Hic-5 at a gene level include, but not limited thereto, e.g., various hybridization methods (e.g., in situ hybridization) using a nucleic acid molecule specifically hybridizing to the nucleic acid molecule encoding Hic-5 or a unique fragment thereof, Northern blotting, Southern blotting, various PCR methods, or a sequencing using NGS or the like, etc.. Approaches for detecting the expression of Hic-5 at a protein level include, but not limited thereto, e.g., an immunoprecipitation method using a substance that specifically recognize Hic-5, such as an antibody, EIA (enzyme immunoassay) (e.g., ELISA (enzyme-linked immunosorbent assay)), RIA (radioimmunoassay) (e.g., IRMA (immunoradiometric assay), RAST (radioallergosorbent test), and RIST (radioimmunosorbent test)), Western blotting, an immunohistochemical method, an immunocytochemical method, flow cytometry, MRI, and the like.

The degree of expression of Hic-5 can be appropriately determined in accordance with each detection approach. The degree of expression of Hic-5 can be determined using, e.g., the reaction intensity of a label attached to a probe in in situ hybridization, the density of a band in Northern blotting, Southern blotting, or Western blotting, the amount of an amplification product in PCR, the number of counts of a target sequence in sequencing, the amount (turbidity, etc.) of precipitates in immunoprecipitation, absorbance or radioactivity in EIA, the area or stain intensity of a stained moiety in immunohistochemical method or immunocytochemical method, or fluorescence intensity or the number of positive cells in flow cytometry.

The greater degree of expression of Hic-5 in the tissue suspected of having the disease than the degree of expression of Hic-5t in the normal tissue may be an increase of 5% or more, 10% or more, 15% or more, 20% or more, 25% or more, 30% or more, 35% or more, 40% or more, 45% or more, 50% or more, 55% or more, 60% or more, 65% or more, 70% or more, 75% or more, 80% or more, 85% or more, 90% or more, 95% or more, or 100% or more, etc.

In one embodiment, the disease detection method of the present disclosure does not include a physician's action. In another embodiment, the disease detection method of the present disclosure is performed in vitro.

The disease detection method of the present disclosure can be performed in combination with the treatment method of the present disclosure or the vascular invasion suppression method of the present disclosure. For example, the treatment method of the present disclosure or the vascular invasion suppression method of the present disclosure can be applied to a subject in which the disease has been detected by the disease detection method of the present disclosure. Thus, one embodiment of the present disclosure relates to a method comprising:
detecting the expression of Hic-5 in a tissue of a subject suspected of having a disease selected from cardiac hypertrophy, interstitial pneumonia, osteoarthritis, fatty liver, and cancer with a risk of vascular invasion;
comparing the degree of expression of Hic-5 in the tissue with the degree of expression of Hic-5 in a normal tissue;
determining that the subject has the disease when the degree of expression of Hic-5 in the tissue suspected of having the disease is greater than the degree of expression of Hic-5 in the normal tissue; and
administering an effective amount of a Hic-5 inhibitor or a composition comprising this inhibitor to the subject determined as having the disease.

Another embodiment of the present disclosure relates to a composition for use in diagnosing a disease selected from cardiac hypertrophy, interstitial pneumonia, osteoarthritis, fatty liver and cancer with a risk of vascular invasion, comprising a Hic-5 detection reagent

Examples of the Hic-5 detection reagent include reagents for use in approaches of detecting Hic-5. Non-limiting examples of the Hic-5 detection reagent include nucleic acid molecules that can specifically hybridize with a nucleic acid molecule encoding Hic-5, primers that permit PCR amplification of a nucleic acid molecule encoding Hic-5, substances capable of specifically recognizing Hic-5 (e.g., the substances that bind to Hic-5, particularly, the antibodies against the antigen Hic-5 and their antigen binding derivatives, and the Hic-5 binding antibody mimetics, described with respect to the composition A of the present disclosure).

A detectable label may be attached to the Hic-5 detection reagent. Examples of the detectable label include, but are not limited to, e.g., fluorescent labels, luminescent labels, radioactive labels, metal atoms detectable by MRI, etc., and compounds (e.g., complexes) containing a metal atom.

The fluorescent label includes, but not limited to, e.g., Cy^{™} series (such as Cy^{™}2, 3, 5, 5.5, 7), DyLight^{™} series (such as DyLight^{™} 405, 488, 549, 594, 633, 649, 680, 750, 800), Alexa Fluor^{®} series (such as Alexa Fluor^{®} 405, 488, 549, 568, 594, 633, 647, 680, 750), HiLyte Fluor^{™} series (such as HiLyte Fluor^{™} 488, 555, 647, 680, 750), ATTO series (such as ATTO 488, 550, 633, 647N, 655, 740), FAM, FITC, Texas-Red, GFP, RFP and Qdot.

The luminescent label includes, but not limited to, e.g., luminol, luciferin, lucigenin and aequorin.

The metal atom include, but not limited to, e.g., gadolinium(III) (Gd(III)), yttrium-88 (⁸⁸Y), and indium-111 (¹¹¹In), and the metal complex includes complexes of the above metal atom(s) and a ligand such as diethylenetriaminepentacetic acid (DTPA), tetraazacyclododecane-1,4,7,10-tetraacetic acid (DOTA), (1,2-ethanediyldinitrilo) tetraacetate (EDTA), ethylenediamine, 2,2'-bipyridine (bipy), 1,10-phenanthroline (phen), 1,2-bis(diphenylphosphino) ethane (DPPE), 2,4-pentanedione (acac), and ethanedioate (ox). Super-paramagnetic iron oxide (SPIO) and manganese oxide (MnO) can also be used.

The radioactive label includes, but not limited to, e.g., technetium-99m (^{99m}Tc), indium-111 (¹¹¹In), iodine-123 (¹²³I), iodine-124 (¹²³I), iodine-125 (¹²⁵I), iodine-131 (¹³¹I), thallium-201 (²⁰¹T1), carbon-11 (¹¹C), nitrogen-13 (¹³N), oxygen-15 (¹⁵O), fluorine-18 (¹⁸F), copper-64 (⁶⁴Cu), gallium-67 (⁶⁷Ga), krypton-81m (^{81m}Kr), xenon-133 (¹³³Xe), strontium-89 (⁸⁹Sr), yttrium-90 (⁹⁰Y), ¹²³I-IMP, ^{99m}Tc-HMPAO, ^{99m}Tc-ECD, ^{99m}Tc-MDP, ^{99m}Tc-tetrofosmin, ^{99m}Tc-MIBI, ^{99m}TcO4-, ^{99m}Tc-MAA, ^{99m}Tc-MAG3, ^{99m}Tc-DTPA, ^{99m}Tc-DMSA and ¹⁸F-FDG1.

The composition of the present disclosure for use in the detection of a disease may be used in vivo (in vivo imaging) or may be used ex vivo.

### EXAMPLES

Hereinafter, the present invention will be described in more detail with reference to Examples. However, the contents of the present invention are not limited by these examples.

### [Example 1] Involvement of Hic-5 in cardiac hypertrophy

Hic-5 KO mice were subjected to transverse aortic constriction (TAC), and their cardiac change was compared with wild-type mice. Hic-5 KO mice (Hic-5^{-/-}) used were prepared using Cre-loxP system (Kim-Kaneyama et al., J Mol Cell Cardiol. 2011;50(1):77-86). Specifically, homologous recombinant ES cells (C57BL/6-based) were prepared using a targeting vector with all exons of Hic-5 flanked by loxP sequences, and hic-5/flox mice were prepared using the ES cell clones. The obtained hic-5/flox mice were mated with Pgk2-Cre transgenic mice (Kido et al., Dev Growth Differ. 2005;47(1):15-24) to obtain Hic-5 KO mice. The chest of each Hic-5 KO or wild-type mouse (C57BL/6) was opened under inhalation anesthesia with isoflurane to expose the transverse aorta. The transverse aorta was ligated together with a 27-gauge needle using a 6.0 silk suture thread. Then, the needle was removed so that stenosis of 0.4 mm in diameter occurred in the transverse aorta. The chest of each mouse was closed, and the mice were raised for 3 weeks and then euthanized, followed by the harvesting of their hearts. As shown in the results of Figure 1, the hearts of Hic-5 KO mice were much smaller than those of wild-type mice, revealing that cardiac hypertrophy was suppressed.

### [Example 2] Involvement of Hic-5 in interstitial pneumonia

### <Study in BLM interstitial pneumonia model>

Bleomycin (BLM) was administered to Hic-5 KO mice, and change in their lung tissues was compared with wild-type mice. Hic-5 KO mice were prepared in the same manner as in Example 1. The mice underwent oral tracheal intubation and were given 2 mg/kg of BLM (bleomycin sulfate, B1141000, Sigma-Aldrich) dissolved at 2 mg/ml in saline. Control group was given the same amount of saline in the same manner as above. Wild-type mice (C57BL/6) were also treated in the same manner as above. The mice were euthanized 14 days after BLM inoculation, and left lung tissues were harvested, fixed in 10% paraformaldehyde for 24 hours, and then embedded in paraffin to prepare 4 µm sections. The sections were subjected to HE staining or Masson's trichrome staining and analyzed under an optical microscope (Figure 2). The expression of Hic-5 and α-SMA in the lung tissues was evaluated by immunostaining and Western blotting. The immunostaining was performed as follows: frozen sections were prepared from the remaining portions of the left lung tissues, washed with PBS, then blocked with a blocking reagent (X0909, Agilent Technologies, Inc.), and stained with an anti-Hic-5 antibody (1:200, 611165, BD Biosciences) or an anti-α-SMA antibody (1:200, ab5694, abeam). Subsequently, detection was performed using fluorescently labeled secondary antibody (anti-mouse IgG Alexa 488 (A11001, Thermo Fisher Scientific) or anti-rabbit IgG Alexa 568 (A11011, Thermo Fisher Scientific)) (Figure 3). Western blotting was performed as follows: right lung tissues harvested from the mice were homogenized in RIPA buffer (10mM Tris (pH7.4), 150mM NaCl, 0.5% NP-40) supplemented with Protease Inhibitor Cocktail (P8340, Sigma-Aldrich) to prepare lung tissue lysates. The obtained lung tissue lysates were separated by SDS-PAGE, transferred to polyvinylidene difluoride membranes (GE Healthcare), and blocked with 0.5% BSA in PBS containing 0.1% Tween 20. Subsequently, the membranes were blotted with an anti-Hic-5 antibody (1:1000) and an anti-α-SMA antibody (1:200) and incubated with HRP-conjugated secondary antibodies. Then, positive bands were visualized by using Western Lightning chemiluminescence reagent (PerkinElmer), followed by exposure to X-ray film (FUJIFILM Corp). The band densities were measured using CS Analyzer 4 (ATTO Corp.) and normalized against GAPDH (Figures 4 and 5).

As is evident from the results of Figure 3, the expression of Hic-5 was induced by the administration of BLM. As shown in Figures 4 and 5, lung tissue fibrosis ascribable to BLM administration was markedly suppressed in Hic-5 KO mice compared with wild-type mice.

### <Expression of Hic-5 in interstitial pneumonia patient>

The expression of Hic-5 was evaluated in lung tissues of an interstitial pneumonia patient (60-year-old male, Japanese). Control normal tissues used were from normal portions of lung tissues of a lung cancer patient (75-year-old female, Japanese, lung adenocarcinoma). Paraffin sections were prepared from the tissues of each subject, deparaffinized with xylene, then washed with ethanol and water, and infiltrated with PBS. Subsequently, the antigen was retrieved using an antigen retrieval agent (Antigen Retrieval Solution, Nichirei Biosciences Inc.), blocked with a blocking reagent (X0909, Agilent Technologies, Inc.), and then stained with an anti-Hic-5 antibody (1:200) or an anti-α-SMA antibody (1:200), followed by color development using a HRP-labeled anti-mouse IgG antibody or anti-rabbit IgG antibody, and DAB. As is evident from the results shown in Figure 6, Hic-5 was highly expressed in the lung tissues of the interstitial pneumonia patient. The results combined with the results obtained in the BLM interstitial pneumonia model mice suggest that human interstitial pneumonia is also suppressed by the suppression of Hic-5.

### <Localization of Hic-5 in lung tissue affected by interstitial pneumonia>

The paraffin sections of wild-type mice obtained in the preceding paragraph "Study in BLM interstitial pneumonia model" were deparaffinized with xylene, then washed with ethanol and water, and infiltrated with PBS. Subsequently, the antigen was retrieved using an antigen retrieval agent (Antigen Retrieval Solution, Nichirei Biosciences Inc.), blocked with a blocking reagent (X0909, Agilent Technologies, Inc.), and then stained with an anti-Hic-5 antibody (1:200) or an anti-α-SMA antibody (1:200), followed by color development using a HRP-labeled anti-mouse IgG antibody or anti-rabbit IgG antibody and DAB. From the results shown in Figure 7, the expression of Hic-5 was found in peribronchiolar Clara cells (a-SMA-negative).

### [Example 3] Involvement of Hic-5 in osteoarthritis

### <Study on osteoarthritis in Hic-5 KO mouse>

Hic-5 KO mice were prepared in the same manner as in Example 1. Osteoarthritis (OA) was induced in accordance with the method described in Kamekura et al., Osteoarthritis Cartilage. 2005;13(7):632-41. Specifically, under general anesthesia, resection of the medial collateral ligament and the medial meniscus of each Hic-5 KO or wild-type mouse was performed under a surgical microscope. After surgery, all mice were maintained under the same conditions and analyzed 8 weeks later. OA severity was quantified using the OARSI system (Glasson et al., Osteoarthritis Cartilage. 2010;18 Suppl 3:S17-23). Specifically, an observer (blinded) exposed mouse knee joints, assigned a score from 0 to 6 to degrees of joint damage in four areas of the medial tibial plateau (MTP), the medial femoral condyle (MFC), the lateral tibial plateau (LTP), and the lateral femoral condyle (LFC), and calculated the summed scores for the four areas (Figure 8, n = 11). The scores of each group were applied to the Student's t-test (unpaired, two-tailed), and P < 0.05 was considered significantly different.

Histological study on joints was conducted by safranin-O staining and scanning electron microscopy. Safranin-O staining was performed in accordance with a standard method. Evaluation under a scanning electron microscope was performed in accordance with the approach described in Arita-Okubo et al., Cardiovasc Res. 2015;105(3):361-71. Specifically, articular cartilages were harvested from posterior aspects of left knee joints of the mice 8 weeks after surgery, followed by immersion fixation with 2.5% glutaraldehyde at 4°C for 24 hours. The samples thus fixed were washed with phosphate-buffered saline (PBS) and stained with 1% osmium tetroxide in a 0.1 M phosphate buffer. Then, the samples thus stained were dehydrated through an ethanol gradient, freeze-dried, coated with platinum, and observed under a scanning electron microscope (S-4700, Hitachi, Ltd.) at 25 kV. As is evident from the results shown in Figures 9 and 10, the joints of Hic-5 KO mice had milder cartilage damage than that in wild type.

### <Progression of osteoarthritis and changes in Hic-5 expression>

The progression of osteoarthritis and changes in Hic-5 expression in articular cartilages were studied. Resection of the medial collateral ligament and the medial meniscus of each wild-type C57BL/6 mouse was performed in the same manner as above. The mice were euthanized before surgery or 2, 4 or 8 weeks after surgery, and knee joint tissues were harvested. The tissues were fixed in 4% paraformaldehyde buffered with PBS at 4°C for 1 day, decalcified with 10% EDTA (pH 7.4) at 4°C for 2 weeks, and then embedded in paraffin. Sagittal sections (4 µm thick) were cut from the specimens and stained with an anti-Hic-5 antibody (1:200) and DAPI. The results shown in Figure 11 revealed that Hic-5 was expressed from 2 weeks after surgery.

### <Localization of Hic-5 in chondrocyte>

Resection of the medial collateral ligament and the medial meniscus of each Hic-5 KO or wild-type mouse was performed in the same manner as above. After surgery, all mice were maintained under the same conditions, and chondrocytes were harvested 8 weeks later. Specifically, the femoral head, the femoral condyle, and the tibial plateau were harvested from euthanized mice using a scalpel. These pieces of cartilage were incubated twice for 45 minutes each in a solution of D-MEM (low glucose, FUJIFILM Wako Pure Chemical) containing 3 mg/mL collagenase D (F. Hoffmann-La Roche, Ltd.) and then incubated overnight in D-MEM (low glucose) containing 0.5 mg/mL collagenase D. On the next day, the collagenase D solution containing residual cartilage was retrieved, and this solution was successively passed through pipettes to disperse cell aggregates. The obtained cell suspension was filtered through a strainer to isolate chondrocytes. The obtained chondrocytes were fixed in 3.7% formaldehyde buffered with PBS, permeabilized with 0.2% Triton-X buffered with PBS, and then blocked with BSA (A6003, Sigma-Aldrich Co., LLC). Then, the cells were stained with an anti-Hic-5 antibody (1:200) and DAPI (n = 3). The results shown in Figure 12 revealed that Hic-5 was localized in focal adhesions.

### <Expression of catabolic factor in joint affected by osteoarthritis>

Resection of the medial collateral ligament and the medial meniscus of each Hic-5 KO or wild-type mouse was performed in the same manner as above. After surgery, all mice were maintained under the same conditions and euthanized 8 weeks later, and knee joint tissues were harvested. The tissues were fixed in 4% paraformaldehyde buffered with PBS at 4°C for 1 day, decalcified with 10% EDTA (pH 7.4) at 4°C for 2 weeks, and then embedded in paraffin. Sagittal sections (4 µm thick) were cut from the specimens and stained with an anti-ADAMTSS antibody (1:50, D-16, Santa Cruz Biotechnology), an anti-type II collagen antibody (1:200, LB-1297, LSL), an anti-type X collagen antibody (1:200, LB-0092, LSL) and an anti-MMP13 antibody (1:100, 18165-1-AP, Proteintech) followed by color development with CSA II, Biotin-Free Catalyzed Amplification System(Agilent). The same sections as above were stained with safranin-O. As shown in Figure 13, the expression of MMP13, ADAMTS5 and type X collagen in Hic-5 KO mice was suppressed as compared with wild-type mice, whereas the expression of type II collagen was higher in Hic-5 KO mice than in wild-type mice. As is evident therefrom, the suppression of osteoarthritis in Hic-5 KO mice was associated with decreased expression of catabolic factors such as MMP13 and ADAMTSS.

### <Influence of inflammatory cytokine on expression of Hic-5 and catabolic factor>

How inflammatory cytokines involved in osteoarthritis would influence the expression of Hic-5 and catabolic factors and how the expression of catabolic factors would be influenced by KO of Hic-5 were studied. Primary articular chondrocytes were isolated from 5-day-old Hic-5 KO mice and wild-type mice in accordance with the approach described in Stanton et al., Nat Protoc. 2011;6(3):388-404. Specifically, the femoral head, the femoral condyle, and the tibial plateau were harvested from euthanized mice using a scalpel. These pieces of cartilage were incubated twice for 45 minutes each in D-MEM (low glucose) containing 3 mg/mL collagenase D (F. Hoffmann-La Roche, Ltd.) and then incubated overnight in D-MEM (low glucose) containing 0.5 mg/mL collagenase D. On the next day, the collagenase D solution containing residual cartilage was retrieved, and this solution was successively passed through pipettes to disperse cell aggregates. The obtained cell suspension was filtered through a strainer and then seeded on a culture dish. Primary chondrocytes thus obtained were maintained in a maintenance medium (D-MEM (low glucose) supplemented with 10% fetal bovine serum (Thermo Fisher Scientific)). The primary chondrocytes were transferred to serum-free DMEM medium and incubated for 24 hours. Subsequently, TNF-a (201-13461, FUJIFILM Wako Pure Chemical) or IL-1β (094-04681, FUJIFILM Wako Pure Chemical) was added at 10 ng/mL to the cultures and incubated for 12 hours or 24 hours. The expression of Hic-5 was evaluated by Western blotting, and the expression of MMP13 was evaluated by real-time qRT-PCR and Western blotting.

The evaluation by Western blotting was performed as follows: chondrocytes were washed with PBS and then lysed in 1% SDS. The obtained lysates were separated by SDS-PAGE, transferred to polyvinylidene difluoride membranes (GE Healthcare), and blocked with 0.5% BSA in PBS containing 0.1% Tween 20. Subsequently, the membranes were blotted with an anti-Hic-5 antibody (1:1000), an anti-GAPDH antibody (1:2000, 171-3, MBL) and an anti-MMP13 antibody (1:400, MAB13426, Merck KGaA) and incubated with HRP-conjugated secondary antibodies. Then, positive bands were visualized using Western Lightning chemiluminescence reagent (PerkinElmer), followed by exposure to X-ray film (FUJI FILM Corp). The band densities were measured using CS Analyzer 4(ATTO) and normalized against GAPDH. The evaluation by real-time qRT-PCR was performed as follows: chondrocytes were washed with a washing buffer attached to RT-qPCR kit (CellAmp^{™} Direct TB Green^{®} RT-qPCR kit, Takara Bio) and then lysed in a lysis buffer attached to the kit. The obtained chondrocyte lysates were subjected to qRT-PCR with the RT-qPCR kit and ABI7900 real-time PCR detection system (Thermo Fisher Scientific). The primers used are shown below. The expression values were normalized against a housekeeping gene (18s), and fold changes were calculated based on control group using the 2^{-ΔΔ} Ct method.
18s
   Forward: 5'-TAGAGGGACAAGTGCGTTC-3' (SEQ ID NO:20)
   Reverse: 5'-CGCTGAGGCCAGTCAGTCAGTCAGTGT-3' (SEQ ID NO:21)
mmp13
   Forward: 5'-TGATGGGACCTTCTTCTGGG-3' (SEQ ID NO:22)
   Reverse: 5'-CATCCATGGTGTGGGGAAAGTTCTTCT-3' (SEQ ID NO:23)

Figure 14 shows the results on Hic-5 expression. Figures 15 and 16 show the results on MMP13 expression. As is evident from the results of Figure 14, the expression of Hic-5 in the chondrocytes was significantly increased by the addition of TNF-oc or IL-1β. As is also evident from the results of Figures 15 and 16, the expression of MMP13 was markedly increased by the addition of TNF-a or IL-1β, whereas the degree of such increase in the chondrocytes was significantly smaller in Hic-5 KO mice than in wild-type mice.

### <Influence of mechanical stress on expression of Hic-5 and catabolic factor>

How mechanical stress, one of the causes of osteoarthritis, would influence the expression of Hic-5 and catabolic factors and how the expression of catabolic factors would be influenced by KO of Hic-5 were studied. Primary articular chondrocytes obtained in the same manner as above were seeded on silicon stretch chambers (culture surface area: 2 cm × 2 cm) coated with collagen at a density of 2.5 × 10⁴ cells/chamber. After culturing for 48 hours, cyclic tensile strain (0.5 Hz, 10% elongation) was applied for 30 minutes using a uniaxial stretching system (NS-550, Scholertec) at 37°C and 5% CO₂ to give the cells mechanical stress. Negative control used was cells cultured under the same conditions as above except that no cyclic tensile strain was applied. The cells were collected 12 hours after stretching. In the same manner as above, the expression of Hic-5 was evaluated by Western blotting, and the expression of MMP13 was evaluated by real-time qRT-PCR and Western blotting. The expression of ADAMTSS was also evaluated by the same real-time qRT-PCR as above using the following primers.
adamts5
Forward: 5'-GCCATTGTAATAACTCCGCACC-3' (SEQ ID NO:24)
Reverse: 5'-TCAGTCAGTGTGGGGAAAGTTCTTCTGG-3' (SEQ ID NO:25)

Figure 17 shows the results on Hic-5 expression. Figures 18 and 19 show the results on MMP13 expression. Figure 20 shows the results on ADAMTS5 expression. As is evident from the results of Figure 17, the expression of Hic-5 in the chondrocytes was significantly increased by the application of mechanical stress. As is also evident from the results of Figures 18 to 20, the expression of MMP13 and ADAMTS5 was markedly increased by the application of mechanical stress, whereas the degree of such increase in the chondrocytes was significantly smaller in Hic-5 KO mice than in wild-type mice.

These results suggested that: in the progression of osteoarthritis, the expression of catabolic factors, such as MMP13 and ADAMTSS, involved in cartilage tissue degeneration is increased with increase in Hic-5 expression; and the expression of the catabolic factors and by extension, cartilage tissue degeneration in osteoarthritis can be suppressed by the suppression of Hic-5 expression.

### [Example 4] Involvement of Hic-5 in vascular invasion of cancer cell

The expression of Hic-5 in lung tissues of a lung adenocarcinoma patient was evaluated by immunostaining. Paraffin sections were prepared from the lung tissues of the patient, deparaffinized with xylene, then washed with ethanol and water, and infiltrated with PBS. Subsequently, antigen was retrieved using an antigen retrieval agent (Antigen Retrieval Solution, Nichirei Biosciences Inc.), blocked with a blocking reagent (X0909, Agilent Technologies, Inc.), and then stained with an anti-Hic-5 antibody (1:200) or an anti-α-SMA antibody (1:200), followed by color development using a HRP-labeled anti-mouse IgG antibody or anti-rabbit IgG antibody, and DAB. As shown in Figure 21, the expression of Hic-5 was found not only in the cancer tissue interstitium but also in cancer cells themselves. Hic-5 was highly expressed, particularly, in cancer cells that invaded vascular channels. These results suggest that high expression of Hic-5 in cancer cells is involved in the invasion of the cancer cells in surrounding tissues, particularly, vascular invasion. The results combined with the results of Example 3 indicating that the high expression of Hic-5 is associated with high expression of MMP13 suggest the possibility that the high expression of Hic-5 in cancer cells also brings about high expression of MMP13 and this promotes the invasion of cancer cells.

### [Example 5] Involvement of Hic-5 in NASH

### <Study in induced NASH model>

6-week-old Hic-5 KO mice and wild-type mice (C57BL/6) were fed with choline-deficient diets (A06071302, Research Diets, Inc.) containing 60 kcal% fat and 0.1% methionine for 6 weeks. After blood collection, the mice were euthanized, and liver was harvested, fixed in 10% paraformaldehyde for 24 hours, and then embedded in paraffin to prepare 4 µm sections. The sections were subjected to Azan staining and analyzed under an optical microscope. The results of Azan staining for Hic-5 KO mice and wild-type mice are shown in Figure 22. ALT and AST in the serum of Hic-5 KO mice and wild-type mice were measured. The results are shown in Figure 23.

From the results of Azan staining in Figure 22, liver tissue fibrosis was markedly suppressed in Hic-5 KO mice compared with wild-type mice, though the deposition of fat droplets in the liver tissues was equivalent therebetween. As shown in Figure 23, elevation in the levels of the hepatocellular injury markers ALT and AST was also suppressed in Hic-5 KO mice to about half the levels in wild-type mice.

Further, the expression of α-SMA in liver tissues was evaluated by immunostaining. The immunostaining was performed as follows: frozen sections were prepared from the remaining portions of the liver tissues, washed with PBS, then blocked with a blocking reagent (X0909, Agilent Technologies, Inc.), and stained with an anti-α-SMA antibody (1:200, ab5694, abeam) and DAPI. Subsequently, detection was performed using anti-rabbit IgG Alexa 488(A32790, Thermo Fisher Scientific). Images were obtained under conditions of objective lens: 20×, exposure time: 300ms, bininng: 1. Representative images of the results are shown in Figures 24 and 25. The areas of portions having fluorescence intensity equal to or larger than a threshold were regarded as α-SMA-positive areas. An α-SMA-positive area ratio against the whole area of each image was calculated from five images as to each mouse and averaged. The α-SMA-positive area ratio (%) is shown in a graph in Figure 26. As is evident from Figures 24 to 26, the expression of α-SMA was markedly suppressed in Hic-5 KO mice compared with wild-type mice. These suggested that hepatocellular injury, hepatic stellate cell activation, and liver tissue fibrosis in NASH can be suppressed by the suppression of Hic-5 expression.

The quantification of fibrotic regions stained blue was attempted using the Azan stain sections of the liver tissues of wild-type mice and Hic-5 KO mice. The Azan stain sections of each individual were photographed in 21 fields of view per individual at a 200× magnification so as not to include vessels of the blood vascular system and such that the liver tissues occupied the whole field of view. An area stained blue in each field of view was measured with MetaMorph^{®} imaging software (Molecular Devices, LLC), and the measurement values of each individual were averaged. Normality for two groups of wild-type mice and Hic-5 KO mice (n = 3 each) was confirmed by the Shapiro-Wilk test using Prism8 (version 8.4.0, GraphPad Software) and p values were calculated by the Welch test. As shown in Figure 27, the percentage of regions stained blue was significantly smaller in Hic-5 KO mice than in wild-type mice (p = 0.0361), revealing that fibrosis was suppressed.

### [Example 6] Treatment of cardiac hypertrophy with Hic-5 inhibitor

A Hic-5 inhibitor was studied for its effect on mouse isoproterenol-induced cardiac hypertrophy model. The Hic-5 inhibitor used was siRNA having the following sequences.
Passenger strand: 5'-CCUAUAGCUGGGCAAGUGG(dT)(dT)-3' (SEQ ID NO:28)
Guide strand: 5'-CCACUUGCCCAGCUAUAGG(dT)(dT)-3' (SEQ ID NO:29)

1 g of isoproterenol hydrochloride (Product# I0260, Tokyo Chemical Industry) was dissolved in 3 mL of saline, and ALZET micro-osmotic pump (model 1004, Durect corporation) was packed with the solution so as to attain 30 mg/kg body weight/day. Under isoflurane anesthesia, the pump was subcutaneously transplanted in C57BL/6 wild-type mice (8-week-old, male) (day 0). For treatment group, siRNA dissolved in in vivo-jet PEI (PolyPlus-transfection SAS) was administered from the tail vein at a dose of 2.4 µg/g body weight twice a week for 3 weeks from week 2 (administration day: days 8, 12, 16, 19, 23, and 26). For vehicle control group, only the in vivo-jet PEI solution was administered in the same manner as above. Untreated control group was also established without pump transplantation.

The mice were euthanized on day 30, and heart harvesting and weight measurement were performed to calculate heart/body weight ratios (mg/g). Some pump-transplanted mice and untreated control group were euthanized on day 7 without siRNA or vehicle administration, followed by heart harvesting, weight measurement, and heart/body weight ratio calculation. Significant difference was evaluated by one-way ANOVA and then the Tukey test. The results are shown in Figures 28 and 29. From Figure 28, cardiac hypertrophy occurred on day 7 due to the administration of isoproterenol, whereas the heart of treatment group on day 30 was slightly smaller than that of vehicle control group, revealing that cardiac hypertrophy was suppressed. From Figure 29, the heart/body weight ratio of treatment group on day 30 was significantly lower than that of vehicle control group.

### [Example 7] Treatment of interstitial pneumonia with Hic-5 inhibitor

The effects of a Hic-5 inhibitor on BLM interstitial pneumonia model were studied. The Hic-5 inhibitor used was the siRNA described in Example 6. C57BL/6 wild-type mice underwent oral tracheal intubation and were given 1 mg/kg of BLM (bleomycin sulfate, B1141000, Sigma-Aldrich) dissolved at 2 mg/ml in saline (day 0). For treatment group, siRNA dissolved in in vivo-jet PEI (PolyPlus-transfection SAS) was administered from the tail vein at a dose of 2.0 µg/g body weight twice a week for 3 weeks from week 2 (administration day: days 9, 13, 17, 20, 23, and 27). For vehicle control group, only the in vivo-jet PEI solution was administered in the same manner as above. The mice were euthanized on day 30, and left lung tissues were harvested, fixed in 10% paraformaldehyde for 24 hours, and then embedded in paraffin to prepare 4 µm sections. The sections were subjected to Masson's trichrome staining and analyzed under an optical microscope to determine Ashcroft scores. Pretreatment control group was euthanized on day 8 after BLM administration, followed by the same evaluation as above. Significant difference was evaluated by one-way ANOVA and then the Tukey test. As is evident from the results shown in Figures 30 and 31, fibrosis was significantly suppressed in treatment group compared with vehicle control group.

### [Example 8] Treatment of osteoarthritis with Hic-5 inhibitor

The effects of a Hic-5 inhibitor on osteoarthritis were studied. The Hic-5 inhibitor used was siRNA having the following sequences.
Passenger strand: 5'-GGAUCAUCUAUACAGCACA(dT)(dT)-3' (SEQ ID NO:30)
Guide strand: 5'-UGUGCUGUAUAGAUGAUCC(dT)(dT)-3' (SEQ ID NO:31)

A siRNA dosing solution was prepared by mixing the siRNA with AteloGene^{®} Local Use "Quick Gelation" (Koken, hereinafter, may be abbreviated as AteloGene^{®} QG). More specifically, a 180 µL aliquot of AteloGene^{®} QG was collected into a 2 mL tube attached to the kit, and 180 µL of QG buffer attached to the kit and 40 µL of a siRNA solution (100µM in nuclease free water) were added thereto. The mixture was stirred at a speed of 12 rpm at approximately 4°C for 10 minutes using a rotator with attention so as not to whip the mixture, followed by centrifugation at 10,000 rpm at approximately 4°C for 1 minute. The obtained supernatant was used as siRNA dosing solution and stored on ice until use. A vehicle dosing solution was prepared in the same manner as in the siRNA dosing solution except that nuclease free water was used instead of the siRNA solution in the same amount.

The medial collateral ligament and the anterior cruciate ligament of the right hindlimb of each Wistar rat (7-week-old, male, Japan SLC) were cut under general anesthesia, and the meniscus was separated off from the femur and the tibia to remove the meniscus (day 0). After surgery, all rats were maintained under the same conditions, and 40 µL of the siRNA dosing solution or the vehicle dosing solution was administered into right knee joints on days 10, 13 and 16 using a syringe (Myjector 29G, 0.5 mL syringe, Terumo Corporation). The rats of siRNA administration group, vehicle control group, and untreated control group given neither siRNA nor vehicle were euthanized on day 19, and the tibia was harvested. In order to evaluate lesions at the start of administration, some rats were euthanized on day 10, and the tibia was harvested (preadministration control group). The harvested tissues were dipped and fixed in a 10% neutral buffered formalin solution, then embedded in paraffin, and sliced to prepare paraffin sections. Each section was stained with safranin-O in accordance with a standard method. OA severity was scored on the basis of the following grades 0 to 6 (depth) and stages 0 to 4 (spread) (Pritzker et al., Osteoarthritis Cartilage. 2006;14(1):13-29).
Grade 0: Normal articular cartilage.
Grade 1: Superficial fibrillation, or death, proliferation. Mid zone and deep zone are normal.
Grade 2: Localized fibrillation from superficial zone to mid zone, death, proliferation, increased/decreased matrix stain.
Grade 3: Fissures and matrix fibrillation extending into mid zone. Branched fissures reaching deep zone, prominent death and proliferation adjacent to fissures.
Grade 4: Loss of cartilage matrix. Erosion up to deep zone.
Grade 5: Lesion extending from calcified zone to bone. Repair by fibrocartilage.
Grade 6: Progression of bone remodeling.
Stage 0: No change
Stage 1: <10%
Stage 2: 10-25%
Stage 3: 25-50%
Stage 4: >50%

The obtained grade and stage were multiplied to calculate OARSI scores from 0 to 24. For example, when a certain site is given grade 3 and stage 2, the score is 3 × 2 = 6. Representative safranin-O stain images are shown in Figure 32. The results on OARSI scores are shown in Table 1 and Figure 33. Significant difference was evaluated by the Kruskal-Wallis test and then the Dunn test. Articular cartilage tissue damage was found in preadministration control group, indicating that the pathological condition was already developed at the start of administration (day 10). siRNA administration group tended to have a mild pathological condition because the score of siRNA administration group was lower than that of vehicle control group.

**Table 1 OARSI scores (mean) of each group**

| Group | Score |
|---|---|
| Preadministration control group (n=3) | 6.3 |
| Untreated control group (n=3) | 22.7 |
| Vehicle control group (n=8) | 22.4 |
| siRNA administration group (n=8) | 14.5 |

### [Example 9] Treatment of NASH with Hic-5 inhibitor

The effects of a Hic-5 inhibitor on NASH were studied. The Hic-5 inhibitor used was the siRNA described in Example 6. C57BL/6 mice (wild-type, 6-week-old, male) were raised with the choline-deficient diet used in Example 5. A complex of siRNA and Invivofectamine^{®} 3.0 Reagent (Thermo Fisher Scientific) was administered from the tail vein at a dose of 0.5 mg (in terms of the amount of siRNA)/kg body weight/wk for 3 weeks from 3 weeks after the start of feeding. Vehicle control group was given a same amount of Invivofectamine^{®} 3.0 Reagent in a same manner, and untreated control group was given none. After the completion of administration, the mice were euthanized, and liver tissues were harvested, fixed in 10% paraformaldehyde for 24 hours, and then embedded in paraffin to prepare 4 µm sections. The sections were subjected to HE staining and analyzed under an optical microscope, and NAFLD activity scores (NAS) were calculated. In order to evaluate pathological conditions at the start of siRNA administration, the mice were euthanized 3 weeks after the start of feeding with the choline-deficient diet and analyzed by the same histopathological study as above (preadministration control group). NAS was calculated in accordance with the description of Kleiner et al., Hepatology. 2005;41(6):1313-21. Specifically, HE stain images were taken as to each of three items, steatosis, lobular inflammation and hepatocellular injury (ballooning). Each item was scored on the basis of Table 2 below, and the scores of each item were totaled and regarded as NAS. Significant difference was evaluated by the Bonferroni test.

**Table 2 Evaluation criteria of each item of NAS**

| Item | Observed image | Evaluation criteria | Score |
|---|---|---|---|
| Steatosis | Any section Magnification: 5 0 | Ratio of steatosis lesion | |
| | | < 5% | 0 |
| | | 5 to 33% | 1 |
| | | > 33 to 66% | 2 |
| | | > 66% | 3 |
| Lobular inflammation | Sections centered at central vein Magnification:200 | Number of inflammation foci | |
| | | None | 0 |
| | | < 2 | 1 |
| | | 2 to 4 | 2 |
| | | >5 | 3 |
| Hepatocellular injury (Ballooning) | Sections centered at central vein Magnification:200 | Number of ballooning hepatocytes | |
| | | None | 0 |
| | | 2 to 3 | 1 |
| | | >4 | 2 |

Representative HE stain images are shown in Figure 34. The results on NAS are shown in Figure 35. These results revealed that the Hic-5 inhibitor significantly suppresses hepatic steatosis.

### [Example 10] Treatment of tumor with Hic-5 inhibitor

The effects of a Hic-5 inhibitor on tumor were studied. The Hic-5 inhibitor used was the siRNA described in Example 6. The animal model used was azoxymethane (AOM)-induced colorectal cancer model. C57BL/6 mice (wild-type, 7 to 11-week-old, male) were intraperitoneally given AOM (011-20171, FUJIFILM Wako Pure Chemical) dissolved in saline at a dose of 12.5 µg/g body weight once a week over 6 weeks. In this model, tumor is usually developed in the large intestine by no later than 15 weeks after the start of AOM administration. In order to study the effect of the Hic-5 inhibitor on the established colorectal tumor, treatment with the Hic-5 inhibitor was performed from 15 weeks after the start of AOM administration. Specifically, ALZET micro-osmotic pump (model 1004, Durect corporation) was packed with siRNA dissolved in saline so as to attain 0.43 µg/g body weight/day.

The pump was intraperitoneally transplanted in the mice under isoflurane anesthesia (siRNA treatment group, n = 3). For vehicle control group (n = 5), a pump packed with saline was transplanted. The mice were euthanized 5 weeks after pump transplantation, and the large intestine was harvested and macroscopically observed for the status of tumor development. As shown in Figure 36, colorectal tumor was confirmed in all the five individuals of vehicle control group. By contrast, no macroscopic tumor was found in one out of the three individuals of siRNA treatment group, and tumor confirmed in the remaining two individuals was slightly smaller than that in vehicle control group and also had no conspicuous blood vessel (Figure 37). In order to study difference in vascular distribution between siRNA treatment group and vehicle control group, tumor tissues were harvested, and 10 µm frozen sections were prepared, washed with PBS, then blocked with a blocking reagent (X0909, Agilent Technologies, Inc.), and stained with an anti-CD34 antibody (1:50, 119302, BioLegend). Subsequently, the specimens were labeled with a fluorescently labeled secondary antibody (anti-rat IgG Alexa 568 (A11077, Thermo Fisher Scientific)), then stained with DAPI, and observed under a fluorescence microscope. As shown in Figure 38, the tumor tissues of vehicle control group had a wider CD34-positive region than that of the tumor tissues of siRNA treatment group, suggesting that blood vessels were densely distributed.

Those skilled in the art will understand that various changes or modifications can be made in the present invention without departing from the spirit of the present invention. Thus, it should be understood that the embodiments of the present invention described herein are given for mere illustration and do not intend to limit the scope of the present invention.

## Claims

1. A composition for use in the treatment of a disease selected from cardiac hypertrophy, interstitial pneumonia, osteoarthritis, fatty liver and tumor, comprising a Hic-5 inhibitor.

2. The composition for use according to claim 1, wherein the cardiac hypertrophy is caused by an increased left ventricular load.

3. The composition for use according to claim 1, wherein the cardiac hypertrophy is a compensated cardiac hypertrophy.

4. A composition for use in suppressing a vascular invasion of a cancer cell, comprising a Hic-5 inhibitor.

5. The composition for use according to any one of claims 1 to 4, wherein the Hic-5 inhibitor is selected from an inhibitory nucleic acid molecule, a genome editing molecule, and an anti-Hic-5 antibody or a nucleic acid molecule encoding the antibody.

6. A method of detecting a disease selected from cardiac hypertrophy, interstitial pneumonia, osteoarthritis, fatty liver and cancer with a risk of vascular invasion, comprising:
detecting an expression of Hic-5 in a tissue suspected of having the disease; and
comparing the degree of expression of Hic-5 in the tissue suspected of having the disease with the degree of expression of Hic-5 in a normal tissue,
wherein a greater degree of expression of Hic-5 in the tissue suspected of having the disease than the degree of expression of Hic-5t in the normal tissue indicates the presence of the disease.

7. A composition for use in diagnosing a disease selected from cardiac hypertrophy, interstitial pneumonia, osteoarthritis, fatty liver and cancer with a risk of vascular invasion, comprising a Hic-5 detection reagent.
